# EUROPÄISCHE PATENTSCHRIFT

(11) **EP 1 228 371 B1**
(45) Veröffentlichungstag und Bekanntmachung des Hinweises auf die Patenterteilung: **25.07.2007**
(21) Anmeldenummer: 00977483.7
(22) Anmeldetag: 03.11.2000
(51) Int. Cl.: G01N 33/537, G01N 33/68, G01N 33/569

(54) **VERFAHREN ZUR CHARAKTERISIERUNG UND ZUR AUFTRENNUNG VON MOLEKULAREN ASSOZIATEN**
METHOD FOR CHARACTERISING AND SEPARATING MOLECULAR ASSOCIATES
PROCEDE DE CARACTERISATION ET DE SEPARATION D'ASSOCIES MOLECULAIRES

(30) Priorität: 03.11.1999 DE 19952955
(43) Veröffentlichungstag der Anmeldung: 07.08.2002
(73) Patentinhaber: ACGT Progenomics AG, 06120 Halle (Saale) (DE)
(72) Erfinder: BÖHM, Gerald, 06114 Halle (Saale) (DE); SCHMIDT, Ulrich, West Leederville, WA 6007 (AU)
(74) Vertreter: Reinhard - Skuhra - Weise & Partner
(86) Internationale Anmeldenummer: PCT/EP2000/010877
(87) Internationale Veröffentlichungsnummer: WO 2001/032694

(56) Entgegenhaltungen:
- EP-A- 0 627 626
- WO-A-99/57566
- PALUTKE M ET AL: "FLOW CYTOMETRIC PURIFICATION OF ALZHEIMER'S DISEASE AMYLOID PLAQUE CORE USING THIOFLAVIN T" CYTOMETRY, Bd. 8, Nr. 5, 1987, Seiten 494-499, XP000872269 ISSN: 0196-4763
- WALL JONATHAN ET AL: "Flow cytometric characterization of amyloid fibrils." METHODS IN ENZYMOLOGY, Bd. 309, 1999, Seiten 460-466, XP001006253 1999 Academic Press Inc.; Academic Press Ltd. 525 B Street, Suite 1900, San Diego, CA, 92101-4495, USA; 24-28 Oval Road, London, NW1 7DX, UK ISBN: 0-12-182210-9
- GOLDMANN CLAUDIA ET AL: "Packaging of small molecules into VP1-virus-like particles of the human polyomavirus JC virus." JOURNAL OF VIROLOGICAL METHODS, Bd. 90, Nr. 1, Oktober 2000 (2000-10), Seiten 85-90, XP001006271 ISSN: 0166-0934
- HERCHER ET AL: 'DETECTION AND DISCRIMINATION OF INDIVIDUAL VIRUSES BY FLOW CYTOMETRY' JOURNAL OF HISTOCHEMISTRY AND CYTOCHEMISTRY Bd. 27, Nr. 1, Januar 1979, Seiten 350 - 352, XP008033612
- MARIE ET AL APPLIED AND ENVIRONMENTAL MICROBIOLOGY Bd. 65, Nr. 1, Januar 1999, Seiten 45 - 52
- STEEN H B: "FLOW CYTOMETRY AND SORTING, SECOND EDITION. XII+824P" 1990, MELAMED, M. R., T. LINDMO AND M. L. MENDELSOHN (ED.) , NEW YORK, NEW YORK, USA; CHICHESTER, ENGLAND, UK. ILLUS , XP008033623 * Seite 605 - Seite 622 *

## Beschreibung

Die vorliegende Erfindung betrifft ein Verfahren zur Charakterisierung und wahlweise zur Auftrennung von molekularen Assoziaten, in einem größenbereich von 1-1000 nm, insbesondere auch solcher Teilchen einer Größe kleiner als 300 nm, wobei mit fluoreszierenden Farbstoffen markierte Teileinheiten der molekularen Assoziate als Marker verwendet werden, und die Charakterisierung der markierten Assoziate oder Aggregate mittels eines FACS (Fluorescence-Activated Cell Sorter) erfolgt.

### Gebiet der Erfindung und Stand der Technik

Bei einer Reihe von Erkrankungen kommt es zur Bildung von morphologisch "unregelmäßigen Assoziaten", das heißt, Aggregaten. Solche pathologischen Ablagerungen bestehen oft aus Proteinen, Proteinfragmenten oder Peptiden, die im Körper verteilt (systemisch) oder in bestimmten Organen wie Pankreas oder im Zentralnervensystem konzentriert gefunden werden. So wird bei der Alzheimer-Erkrankung als charakteristische Ablagerung im Gehirn ein Peptid (Alzheimer-β-Peptid, Länge meist 42 Aminosäuren, als Fragment eines größeren Vorläuferproteins) gefunden, das sogenannte amyloidogene Aggregate ("senile Plaques") ausbildet. Daneben tritt bei der Alzheimer-Erkrankung als zweites Merkmal die Ausbildung von neurofibrillären Strukturen ("tangles" oder "paired helical filaments") auf, die von dem Tau-Protein gebildet werden. Bis heute ist bei vielen amyloidogenen Erkrankungen nicht abschließend geklärt, inwiefern diese Proteinablagerungen in den Krankheitsverlauf eingreifen, und ob diese Ablagerungen kausal für die Erkrankung verantwortlich sind oder lediglich Begleiterscheinungen darstellen.

Ein ähnliches Bild zeigen die transmissiblen spongiformen Enzephalopathien, die durch das Prion-Protein induziert werden. Deren menschliche Ausprägungsform findet sich in der Creutzfeld-Jakob-Erkrankung, im Tierreich sind besonders die Erkrankung des Rindes (BSE) und der Schafe (Scrapie) bekannt. Ein möglicher Zusammenhang zwischen dem Auftreten von BSE beim Rind und einer neuen Form der Creutzfeld-Jakob-Erkrankung (vCJD) wird dabei derzeit nicht ausgeschlossen (M.E. Bruce et al., Transmissions to mice indicate that new variant' CJD is caused by the BSE agent, Nature 389, S. 498-501, 1997). Für die analoge Erkrankung des Schafes (Scrapie) wurde beobachtet, daß ein Zusammenhang zwischen der Konzentration krankheitsrelevanter Proteine und der Infektiösität existiert. Die nachfolgende Tabelle ist verändert übernommen und erweitert aus Kelly, J.W., Alternarive conformations of amyloidogenic proteins govern their behavior, Curr. Opin. Struct. Biol. 6, S. 11-17, 1996.

**Vonviegend neurodegenerative amyloidogene Erkrankungen :**

| **Erkrankung** | **Beteiligtes Protein** |
|---|---|
| Morbus Alzheimer | β-Protein/Alzheimer-β-Peptid (1-40, 1-42, 1-43); Tau-Protein |
| Transmissible Spongiforme Enzephalopathie (CJD, Kuru, BSE, Scrapie) | Prion-Protein |
| Chorea Huntington | Huntingtin |
| Morbus Parkinson | Synuclein |
| Vererbbare Cerebrale Amyloide Angiopathie | Cystatin C |

**Andere amyloidogene Erkrankungen:**

| **Erkrankung** | **Beteiligtes Protein** |
|---|---|
| Injektionslokalisierte Amyloidose | Insulin |
| β-2-Mikroglobulin-Amyloidose | β-2-Mikroglobulin |
| Primäre Asystemische Amyloidose | Immunglobulin |
| Finnische Vererbte Systemische Amyloidose | Gelsolin |
| Atriale Amyloidose | Atrial Natriuretic Factor |
| Familiäre Amyloide Polyneuropathie | Transthyretin |
| Medullaria-Carcinom der Schilddrüse | Calcitonin |
| Vererbbare Nichtneuropathische Amyloidose | Lysozym |
| Diabetes mellitus Typ II | Islet-Amyloid-Polypeptid |
| Reaktive Asystemische Amyloidose | Lipoproteine |
| Cleidocraniale Dysplasie | Transkriptionsfaktor CBFA 1 |
| Vererbte Renale Amyloidose | Fibrinogen |
| Okularpharyngeale Muskeldystrophie | Poly(A)-Bindungsprotein II |

Die bei diesen Erkrankungen auftretenden Aggregate werden hauptsächlich aus den angegebenen Proteinen oder aus Fragmenten dieser Proteine gebildet und sind jeweils hoch charakteristisch für das Vorliegen der Erkrankung. Potentielle Diagnostik-Verfahren dieser Erkrankungen können somit darauf beruhen, daß die Proteine bzw. Proteinfragmente, das heißt, Teileinheiten der molekularen Assoziate unter geeigneten Bedingungen auch *in vitro* zu einer selektiven Aggregation bzw. Zusammenlagerung neigen. In den meisten Fällen der genannten Erkrankungen ist ein diagnostischer Nachweis bisher jedoch schwierig oder technisch noch ungelöst. Bisher bekannte Testverfahren zur Alzheimer-Diagnostik gehen meist von einem immunologischen Nachweis der beteiligten Proteine und Peptide, und damit nicht von einem direkten Nachweis der Assoziate bzw. der aggregierten Ablagerungen oder deren Teilkomponenten aus. Dabei wird dem Patienten in der Regel Cerebrospinalflüssigkeit (CSF, Liquor) durch eine schmerzlose Lumbalpunktion entnommen. In der CSF sind die nachzuweisenden Substanzen für die Alzheimer-Erkrankung enthalten. Eine Präzisierung des Nachweises gelingt dabei durch die gleichzeitige Messung der beiden löslichen Alzheimer-spezifischen Substanzen, Tau-Protein und Amyloid-β-Peptid (M. Shoji et al., Combination assay of CSF tau, Aβ1- 40 and Aβ1-42(43) as a biochemical marker of Alzheimer's disease, J. Neurol. Sci. 158, S. 134-140, 1998; F. Hulstaert, K. Blennow, A. Ivanoiu, H.C. Schoonderwaldt, M. Riemenschneider, P.P. De Deyn, C. Bancher, P. Cras, J. Wiltfang, P.D. Mehta, K. Iqbal, H. Pottel, E. Vanmechelen, und H. Vanderstichele: Improved discrimination of AD patients using beta-amyloid (1-42) and tau levels in CSF, Neurology 52, S. 1555-1562, 1999).

Im U.S.-Patent 5,593,846 ist ein Verfahren zur Bestimmung der Konzentration des gelösten Amyloid-β-Peptides beschrieben, die pathologische Komponente (Ablagerungen) wird hierbei jedoch nicht erfaßt.

Im U.S.-Patent 5,434,050 wird ein Diagnoseverfahren für Alzheimer beschrieben, bei dem das Peptid an feste Strukturen (beispielsweise Hirnbiopsiematerial) gebunden wird. Ein solches Verfahren kann nicht ohne schwerwiegenden medizinischen Eingriff erfolgen und wird derzeit nicht angewandt.

Im Patent WO 99/15903 ist ein Verfahren beschrieben, wie pathologische Ablagerungen mit Hilfe der FCS-Methode (Fluorescence Correlation Spectroscopy) nachgewiesen werden können. Die FCS-Methode ist jedoch diffusionskontrolliert und technisch nicht für hohen Durchsatz geeignet, es können nur wenige, maximal 2 bis 3 molekulare Spezies verschiedener Größe unterschieden werden, wobei die Spezies mindestens um eine Zehnerpotenz unterschiedliche Massen besitzen müssen und die Größenverteilung nur abschätzbar-ist, und es können in jedem Verfahrensschritt nur jeweils wenige (in der Regel ein) Fluoreszenz-Farbstofftyp(en) eingesetzt werden. Die Selbstaggregation der Sonde ist dabei ein signifikantes Verfahrensproblem. Die Methode ist daher nicht geeignet, ein breites Spektrum von möglichen pathologischen Signalen und Ausprägungen eindeutig zu erfassen, insbesondere wird eine parallele Bestimmung von β-Peptid und Tau-Protein nicht erreicht. Ebenfalls ist der zeitliche Aufwand pro Messung erheblich. Im Patent WO 99/15903 ist als wesentliches Merkmal die zeitliche Einschränkung angegeben, dass die Assoziation der Sonde an das Target gemessen wird, bevor eine Selbstaggregation der Sonde überwiegt.

Das U.S.-Patent 5,486,460 beschreibt ein Verfahren zur Alzheimer-Diagnostik, bei dem aufkonzentrierte Cerebrospinalflüssigkeit getrocknet wird und anschließend mit Thioflavin S angefärbt wird. Dieses Verfahren ist jedoch in der praktischen Handhabung äußerst unattraktiv und ist auch nicht ausreichend spezifisch für eine klinische Diagnose, daher findet das Verfahren keine weitere Beachtung.

Das Patent WO 9 704 311 A2 beansprucht ein FACS-basiertes Vefahren zur Isolierung lebender Zellen aus einer Mischung verschiedener lebender Zellen, die sich in Anwesenheit und Verteilung auf ihrer Oberfläche befindlicher Rezeptoren unterscheiden. Dabei werden in vorteilhafterweise gegenüber dem Stand der Technik keine fluoreszenzmarkierten Antikörper verwendet, die eine Permeabilisierung und somit ein Abtöten der Zellen erfordern, sondern fluoreszenzmarkierte Peptide, die von natürlichen Liganden auf der Zelloberfläche vorhandener Rezeptoren abgeleitet sind. Bei einer Inkubation der Zellen mit derartigen Peptiden lagern sich diese an ihre entsprechenden Rezeptoren an und markieren somit eine bestimmte Zellpopulation, die mit Hilfe eines FACS isoliert werden kann. Das Verfahren betrifft also letztlich die im Stand der Technik übliche Anwendung des FACS-Verfahrens zur Analyse und Sortierung von Zellen.

Im Patent US-PS 5 540 494 wird eine Methode beschrieben, die es erlaubt, aus Daten, die mit einem konventionellen Durchfluss-Zytometer gemessen wurden, den absoluten Radius und die absolute Oberfläche der analysierten Partikel zu berechnen. Als Größenbereich wird fürdie Partikel 0,33 - 8,98 µm angegeben. Die Charakterisierung kleinerer Partikel ist nicht beschrieben.

Palutke at al., Cytometry 8:494-499, 1987, beschreiben die Bindung von Thioflavin T an Amyloid Plaque Core Proteine (APCP), wobei bekannt wurde, dass Thioflavin T nicht an Lipofuscin bindet. Da APCP-Teilchen häufig mit Lipofuscin-Teilchen kontaminiert sind, bestand die Notwendigkeit, die APCP-Teilchen von dem kontaminierenden Lipofuscin abzutrennen. Gemäß Palutke et al. wurde ein Gemisch aus APCP und Liposfuscin mit Thioflavin T vermischt, und man konnte durch FACS-Analyse eine Aufreinigung von Lipofuscin erreichen.

Hercher et al., The Journal of Histochemistry and Cytochemistry, Band 27, Nr. 1, Seiten 350-352, 1979, beschreiben die Verwendung durchflusscytometrischer Verfahren zur Charakterisierung individueller Virusteilchen. Die Virusteilchen werden mit fluoreszenzmarkierten Antikörpern markiert und mit Hilfe von Durchflusscytometrie analysiert.

Es wäre in jedem Falle höchst wünschenswert, ein hochspezifisches und sensitives Verfahren zu entwickeln, mit dem die mit den genannten Krankheiten verbundenen Peptide (Proteinfragmente) in löslicher Form oder auch in Form von - ablagerungsbildenden - Kristallisationskeimen nachgewiesen werden können und somit eine eindeutige und empfindliche Diagnose der jeweiligen Krankheit erfolgen kann. Es wäre weiterhin enorm vorteilhaft, möglichst frühzeitig eine korrekte (biochemische, serologische) Diagnostik der Erkrankung vornehmen zu können, um noch vor Ausbruch der Erkrankung erste therapeutische Schritte einzuleiten. Die Pflegekosten für Alzheimer-Erkrankte sind beträchtlich. Eine frühzeitige Diagnose, verbunden mit einer nachfolgenden erfolgreichen Therapie, würde somit zu erheblichen Einsparungen im Gesundheitswesen führen. Darüberhinaus könnte eine solche Diagnostik als Vorsorgeuntersuchung regelmäßig auch bei gesunden bzw. unverdächtigen Personen fortgeschrittenen Alters als Vorbeugemaßnahme angewandt werden.

Aufgabe der vorliegenden Erfindung ist es daher, ein Verfahren zur Charakterisierung von molekularen Assoziaten bereitzustellen, das die genannten Nachteile des Stands der Technik nicht aufweist.

Dies wird erfindungsgemäß durch ein Verfahren nach Anspruch 1 zur Charakterisierung von aus Teileinheiten bestehenden molekularen Assoziaten erreicht, wobei:
- nicht assoziierte Teileinheiten mit mindestens einem fluoreszierenden Farbstoff markiert werden,
- die markierten Teileinheiten untereinander oder mit nicht markierten Teileinheiten oder mit aus Teileinheiten bestehenden molekularen Assoziaten in Kontakt gebracht werden, um eine Anlagerung und/oder Bindung der markierten Teileinheiten aneinander oder an nicht markierte Teileinheiten oder an die aus Teileinheiten bestehenden molekularen Assoziate zu erreichen, um markierte molekulare Assoziate zu bilden,
und
- die markierten molekularen Assoziate mittels eines FACS (Fluorescence-Activated Cell Sorter) charakterisiert werden,
und
- die zu charakterisierenden molekularen Assoziate einen Größenbereich von 1 bis 1000 nm aufweisen, und
- die molekularen Assoziate Viren- oder Phagenkapside, Proteasomen, Chaperon-Komplexe oder Ribosomen sind oder aus modifizierten Teileinheiten hiervon zusammengesetzt sind, oder
- aus gleichen Teileinheiten bestehende Peptid- oder Protein-Assoziate, einzelsträngige oder doppelsträngige DNA, einzelsträngige oder doppelsträngige RNA, Lipid-Assoziate, Phospholipid-Assoziate, Kohlenhydrat-Assoziate, Polysaccharid-Assoziate, Assoziate enthaltend Kohlenwasserstoff-Verbindungen mit hydrophilem oder hydrophobem Charakter, Assoziate aus isoprenoiden Verbindungen, oder Gemische hiervon sind.

Vorteilhafte Ausführungsformen der Erfindung ergeben sich aus den Unteransprüchen sowie der Beschreibung.

### Beschreibung

Im Rahmen von Untersuchungen der biochemischen, biotechnologischen, und medizinischen Diagnostik stellt sich regelmäßig das Problem, Assoziate molekularer Substanzen zu charakterisieren. Die Teileinheiten der Assoziate können dabei unterschiedlichen chemischen Klassen angehören, wie Peptiden bzw. Proteinen, Nukleinsäuren, Lipiden und Phospholipiden. Kohlenhydraten und Polysacchariden sowie davon abgeleitete Substanzen, oder die Assoziate können auch Teileinheiten aus verschiedenen Klassen enthalten. Solche Charakterisierungen sind für medizinisch-diagnostische oder therapeutische Anwendungen relevant. Auch in den biowissenschaftlichen und chemischen Grundlagenforschungen sowie der anwendungsbezogenen Forschung können solche Charakterisierungen erforderlich sein.

In der vorliegenden Erfindung wird ein unerwartetes experimentelles Ergebnis dazu eingesetzt, solche molekularen Assoziate zu vermessen und zu charakterisieren, sowie ein Ensemble aus solchen Zusammenlagerungen bezüglich ausgewählter Eigenschaften wie Größe oder Zusammensetzung aufzutrennen.

Überraschenderweise wurde gefunden, daß die Charakterisierung von molekularen Assoziaten durch eine Methode gelöst werden kann, die ursprünglich für die Charakterisierung und Auftrennung von Zellen entwickelt wurde. Die dazu verwendeten Geräte nennen sich demzufolge FACS-Geräte (Fluorescence-Activated Cell Sorter). Ein FACS-Gerät (Zellsortiergerät, in einer einfacheren Version auch als Durchfluß-Zytometer bekannt) ist, vereinfacht ausgedrückt, ein optisches Meßsystem, das Streulicht- und Fluoreszenzsignale einzelner, in einem Flüssigkeitsstrom fokussierter Teilchen in einem vereinzelten Flüssigkeitströpfchen analysiert. Im Gegensatz zu einem statischen Fluorimeter beruhen die Ergebnisse auf einer gleichzeitigen Messung mehrerer physikalischer Parameter jedes einzelnen Teilchens, das im Flüssigkeitsstrom das Detektionssystem passiert. Die optische Anregung geschieht dabei durch Laser. Die Auswertung erfolgt nach Auszählen einer statistisch gesicherten Anzahl von Einzelereignissen (Partikeln) im Flüssigkeitsstrom. Zellsortiergeräte (FACS-Geräte) haben gegenüber Durchfluß-Zytometern die zusätzliche Möglichkeit, jeden einzelnen partikelhaltigen Tropfen im Flüssigkeitsstrom gemäß seinen gemessenen Eigenschaften wie Fluoreszenzintensität oder Streulichteigenschaften (Größe und Form bzw. Granularität des Partikels) mit einer elektrischen Ladung zu versehen, die nachfolgend zur Sortierung des Partikels in verschiedene Behälter verwendet werden kann. Dabei wird das geladene Tröpfchen mit dem darin enthaltenen Teilchen durch ein elektrisches Feld geführt und entsprechend seiner Ladung abgelenkt.

FACS-Geräte ermöglichen quantitative Messungen an individuellen Partikeln mit hoher Präzision. Insbesondere bieten FACS-Geräte die Möglichkeit, eine große Anzahl an Partikeln in sehr kurzer Zeit zu analysieren. Weiterhin ist vorteilhaft, daß eine Charakterisierung der Partikel im präparativen Maßstab erfolgen kann, da eine Sortierung der Partikel nach bestimmten, vordefinierten Eigenschaften (beispielsweise Größe oder Fluoreszenzintensität) möglich ist. Im FACS-Gerät erfolgt die Analyse von in einem Flüssigkeitsstrom befindlicher Partikel primär hinsichtlich ihrer Streulicht- und Fluoreszenzsignale.

Im Stand der Technik wurden bisher ausschließlich Systeme mit Zellen oder Zellorganellen mit Hilfe von FACS-Geräten vermessen. Überraschenderweise konnte nachgewiesen werden, daß mit FACS-Geräten, bevorzugt mit FACS-Geräten der neuesten Generation, auch einzelne molekulare Assoziate und Aggregate charakterisiert werden können. Diese Assoziate können erfindungsgemäß viel kleiner sein, als die Auflösungsgrenze derartiger Geräte (ca. 300 nm) beträgt. Es kommt durch die Verwendung von geeigneten Fluoreszenzfarbstoffen zu Effekten in der verwendeten Lösung, die eine Charakterisierung der molekularen Substanzen bezüglich Größe und Zusammensetzung mit hoher Reproduzierbarkeit erlauben. Dabei kann jeder Fluoreszenzfarbstoff verwendet werden, der nach der Bildung des molekularen Assoziates ein ausreichend intensives Fluoreszenzsignal für die Detektion des Assoziates abstrahlt. Farbstoffe mit hoher Quantenausbeute sind dabei bevorzugt, wenn die Sensitivität des Verfahrens hoch sein soll. Gleichzeitig können durch das laserbasierte Verfahren anhand der Seitwärtsstreuung Informationen über die Form und Granularität, sowie anhand der Vorwärtsstreuung über die Größe der molekularen Assoziate und Aggregate gewonnen werden.

Die vorliegende Erfindung stellt ein Verfahren zur Charakterisierung von molekularen Assoziaten bereit. Die molekularen Assoziate können dabei aus chemisch verschiedenen oder gleichartigen Teileinheiten, die spezifisch oder unspezifisch zusammengelagert sind, bestehen. Nicht-assemblierte Teileinheiten der molekularen Assoziate werden bei dem erfindungsgemäßen Verfahren mit mindestens einer optischen Markierung, nämlich mit mindestens einem fluoreszierenden Molekül, versehen. Diese markierten Teileinheiten werden anschlie-ßend als "Marker" untereinander oder mit nicht markierten Teileinheiten oder mit aus Teileinheiten bestehenden molekularen Assoziaten in Kontakt gebracht, um eine Anlagerung und/oder Bindung der markierten Teileinheiten aneinander oder an nicht markierte Teileinheiten oder an die aus Teileinheiten bestehenden molekularen Assoziate zu erreichen. Auf diese Weise werden markierte molekulare Assoziate gebildet, die anschließend mit Hilfe eines FACS (Fluorescence-Activated Cell Sorter) bezüglich Größe, Form und Zusammensetzung charakterisiert werden können. Anschließend kann wahlweise eine Auftrennung der untersuchten Assoziate nach an sich bekannten Verfahren durchgeführt werden, beispielsweise eine Auftrennung der molekularen Assoziate gemäß ihrer Größe oder nach ihrer Fluoreszenzsignalintensität. Dabei wird den Assoziaten, die eine bestimmte vorgewählte Eigenschaft (Größe, Signalintensität) besitzen, durch das FACS-Gerät eine elektrische Aufladung zuteil. Durch die elektrische Ladung des Assoziates erfolgt dann nachfolgend eine Auftrennung (Sortierung). Andere Trennmethoden sind dem Fachman bekannt und können je nach chemischer oder physikalischer Beschaffenheit der molekularen Assoziate eingesetzt werden. Nach der Auftrennung kann eine weitere Charakterisierung der sortierten Assoziate erfolgen, beispielsweise durch optische Verfahren wie beispielsweise Messung der Fluoreszenzintensität, Fluoreszenzspektroskopie, Lichtstreuung, Absorptionsspektroskopie und/oder gemäß den circulardichroitischen oder lineardichroitischen Eigenschaften oder der Streulichtverteilung der molekularen Assoziate. Da die Charakterisierung mittels FACS im Durchfluß erfolgt, kann die Auftrennung der molekularen Assoziate direkt im Anschluß an die FACS-Charakterisierung durchgeführt werden.

Bei der Charakterisierung mittels FACS kann mehr als ein Farbstoff gleichzeitig erfaßt werden. Dadurch können bei Verwendung verschiedener Fluoreszenzfarbstoffe zur Markierung verschiedener Teileinheiten der molekularen Assoziate mehrere verschiedene Teileinheiten eines molekularen Assoziats unterschieden werden.

Durch die Charakterisierungen können erfindungsgemäß einerseits Aussagen über die Zusammensetzung der Assoziate und Aggregate selbst getroffen werden (beispielsweise durch die Markierung verschiedener Teileinheiten eines Assoziates oder Aggregates mit verschiedenen Fluoreszenzfarbstoffen). Erfindungsgemäß können bei Verwendung verschiedener Fluoreszenzfarbstoffe für verschiedene Teileinheiten mehrere verschiedene Teileinheiten der molekularen Assoziate voneinander unterschieden werden. Bei dem derzeitigen Stand der Gerätetechnik ist die Unterscheidung von bis zu vier verschiedenen Teileinheiten der molekularen Assoziate möglich. Andererseits können auch Aussagen zur Populationsverteilung von Assoziat- oder Aggregatgemischen getroffen werden, also eine Verteilungsbestimmung von Größe und Form der einzelnen Aggregate und Assoziate in der Suspension oder Lösung vorgenommen werden.

Eine Selbstassoziation der fluoreszenzmarkierten Substanz, ohne Einschluß von der in der Testlösung (beispielsweise Patientenliquor) vorhandenen nichtmarkierten Substanz, kann durch das beschriebene Verfahren von dem Fall unterschieden werden, bei dem wie gewünscht die nichtmarkierte Substanz in der Testlösung in die Assoziate mit eingeschlossen wird. Dies erfolgt durch die vergleichende Bestimmung der molekularen Masse und der Form der Aggregate (durch die Streulichtanteile des FACS-Signals) sowie in Relation dazu die Messung der Fluoreszenzintensität der Aggregate. Aus dem Vergleich der multiparametrischen Datensätze mit entsprechend gewählten Referenzstandards kann die Selbstaggregation der fluoreszenzmarkierten Substanz, also ein Meßartefakt, von einer korrekten Messung unterschieden werden, indem jeweils der Anteil an nichtfluoreszenzmarkierter Substanz in dem vermessenen Aggregat bestimmt wird. Insgesamt zeigt das in der vorliegenden Beschreibung entwickelte Verfahren keine Beeinflussung der Ergebnisse durch die Selbstaggregation der Sonde, da solche Selbstaggregate anhand der Lichtstreuungseigenschaften sowie der Fluoreszenzintensität von heterogenen Assoziaten unterschieden werden kann. Das Zulassen einer Sonden-Selbstaggregation kann für das vorliegende Verfahren hilfreich sein, da in diesem Falle eine optimale Sensitivität erzielt werden kann. Eine zeitliche Einschränkung liegt in der vorliegenden Erfindung daher nicht vor. Der Nachweis bzw. die Charakterisierung der Assoziate kann in einer Ausführungsform dieser Erfindung erfolgt sogar vorteilhafterweise zu einem Zeitpunkt und unter Bedingungen der Selbstaggregation der Sonde durchgeführt werden.

Erfindungsgemäß werden zwei Formen von molekularen Assoziaten unterschieden. Zum einen werden Zusammenlagerungen betrachtet, die zu geometrisch regelmäßigen Strukturen mit einer jeweils weitgehend homogenen Population führen. Solche Zusammenlagerungen werden in dieser Erfindung als "regelmäßige" molekulare Assoziate bzw. als molekulare Assoziate mit räumlich und/oder stöchiometrisch definierter Struktur bezeichnet; sie werden durch spezifische Assoziationsprozesse erreicht. Beispiele für solche molekularen Assoziate sind Virus- oder Phagen-Hüllen (vgl. nachstehend), die gelegentlich aus nur einem Untereinheitentyp aufgebaut sind und oft ikosaedrische Symmetrie besitzen oder aus heterogenen Untereinheiten aufgebaute makromolekulare Assoziate wie Ribosomen, Chaperon-Komplexe oder Proteasomen. Zum anderen werden erfindungsgemäß molekulare Assoziate umfaßt, die eine statistische Verteilung in ihrer Größe und Zusammensetzung besitzen können, und die nicht oder allenfalls teilweise aus regelmäßigen Zusammenlagerungen bestehen. Diese molekularen Assoziate werden erfindungsgemäß als "Aggregate" oder als molekulare Assoziate, die bezüglich ihrer Struktur und/oder Zusammenlagerung uneinheitlich sind, bezeichnet. Solche Aggregate treten beispielsweise bei der rekombinanten Herstellung von Proteinen in Form von *inclusion bodies* auf, oder sie sind pathologische Kennzeichen von Erkrankungen in Form von amyloidogenen Plaques, *inclusion bodies,* oder anderen morphologischen Strukturen. Sie sind meist variabel in Größe und Zusammensetzung. Als genereller Oberbegriff für beide Formen der Zusammenlagerung wird in dieser Erfindung meist der vereinfachende Terminus "molekulare Assoziate" gewählt, die bezüglich ihrer Regelmäßigkeit nicht eingeschränkt sind.

Erfindungsgemäß überdecken die zu charkterisierenden Assoziate einen Größenbereich von 1 - 1000 nm, bevorzugt ist ein Größenbereich von 10 - 300 nm, ebenfalls bevorzugt 10 - 100 nm und weiterhin bevorzugt 1 -100 nm.

Das in der vorliegenden Beschreibung eingesetzte Verfahren erlaubt es beispielsweise, regelmäßige molekulare Assoziate zu charakterisieren. Solche Assoziate finden sich beispielsweise in viralen Hüllstrukturen, die in vielen Fällen ikosaedrisch gestaltet sind. Andere Viren oder Phagen besitzen nichtikosaedrische Symmetrie, sie sind beispielsweise filamentös, helikal, oder besitzen andere morphologische Ausgestaltungsformen. Die Hüllen von Viren und Phagen besitzen in der Regel eine definierte Zusammensetzung, mit präzise zueinander orientierten Untereinheiten, und stellen damit gute Modellsysteme für makromolekulare Assoziate mit regelmäßiger Zusammensetzung und/oder Struktur dar. Eine Charakterisierung von Größe sowie molekularer Zusammensetzung für jede einzelne Virushülle kann eine erhebliche analytische Hilfe bei der Untersuchung dieser Virushüllen sowie auch bei anderen molekularen Assoziaten (wie zellulären Proteasomen, Chaperon-Komplexe, oder Ribosomen) sein. Als Beispiele für solche Viren und Phagen sind - nachfolgend nach ihrer primären Morphologie sortiert - zu nennen:

| Morphologie | Vertreter (Virus bzw. Phage) |
|---|---|
| Amorph bzw. unbekannt | Umbravirus; Tenuivirus |
| Bacilliform | Baculoviridae; Badnavirus; Barnaviridae; Filoviridae; Rhabdoviridae |
| Filamentös | Capillovirus; Carlavirus; Closterovirus; Furovirus; Inoviridae; Lipothrixviridae; Potexvirus; Potyviridae; Tobamovirus; Tobravirus; Polydnaviridae |
| Helikal | Hordeivirus; Paramyxoviridae; Trichovirus |
| Ikosaedrisch | Adenoviridae; Astroviridae; Birnaviridae; Bromoviridae; Caliciviridae; Caulimovirus; Circoviridae; Comoviridae; Corticoviridae; Dianthovirus; Enamovirus; Hepadnaviridae; Herpesviridae; Idaeovirus; Iridoviridae; Lviviridae; Luteovirus; Machlomovirus; Marafivirus; Microviridae; Necrovirus; Nodaviridae; Papovaviridae; Partitiviridae; Parvoviridae; Phycodnaviridae; Picornaviridae; Reoviridae; Rhizidiovirus; Sequiviridae; Sobemovirus; Tectiviridae; Tetraviridae; Tombusviridae; Totiviridae; Tymovirus |
| Isometrisch | Cystoviridae; Geminiviridae |
| Oval | Poxviridae |
| Pleomorph | Coronaviridae; Hypoviridae; Plasmaviridae |
| Sphärisch | Arenaviridae; Arterivirus; Bunyaviridae; Flaviviridae; Orthomyxoviridae; Retroviridae: Togaviridae |
| Zitronenförmig | Fuselloviridae |
| Phagen mit | Myoviridae; Podoviridae; Siphoviridae |
| Schwanzfortsatz | |

Ein Beispiel für solche viralen Hüllstrukturen ist nachstehend anhand des Polyomavirus-Pseudokapsids (aus VP1-Untereinheiten des Polyomavirus bestehende Proteinhülle) gezeigt. Bei den in vorstehender Tabelle dokumentierten Beispielen ist das SSV1-Partikel (Fuseolloviridae) hervorzuheben, das das Archaebakterium *Sulfolobus shibatae* infiziert. Dieser Vertreter der Phagen ist aufgrund seiner Wirtsspezifität hyperthermophil, mithin auch bei hohen Temperaturen stabil und kann daher für eine Vielzahl von Anwendungen im Bereich der Biotechnologie und der Medizin vorteilhaft genutzt werden. Es ist in der Lage, eine sehr stabile Proteinhülle auszubilden. Ähnliche Vertreter finden sich beispielsweise auch bei den Lipothrixviridae. Noch nicht weiter klassifiziert sind die thermophilen und hyperthermophilen Vertreter der Bacilloviridae und der Guttaviridae, die in solchen Prozessen, wo die Stabilität einer Proteinhülle (gebildet aus den Phagenproteinen) relevant ist, eingesetzt werden können.

Fluoreszenzmarkierungen erfolgen oft mit der spezifischen kovalenten Kopplung von Farbstoffen an Thiolgruppen in Proteinen (Cysteine) und anderen molekularen Substanzen. Dazu werden oft reaktive Maleimid-Derivate oder Jodacetamid-Derivate von fluoreszierenden Substanzen eingesetzt. Für die Kopplung an Aminogruppen werden beispielsweise Succinimidylester, Sulfonylhalide, Isothiocyanate, und Aldehyde als Fluoreszenzmarker-Derivate eingesetzt. Weitere Agentien existieren zur spezifischen Kopplung an OH-Gruppen (in Proteinen und Peptiden beispielsweise bei Serin, Threonin, oder Tyrosin), an Aldehyde oder Ketone (beispielsweise zur Markierung von Polysacchariden), und aktivierte Carboxylgruppen. Spezifische Anwendungen können auch in der Kopplung von Effektormolekülen wie fluoreszenzmarkiertem Biotin (Biotinylierung) liegen.

Auch durch nichtkovalente Kopplung kann eine spezifische Fluoreszenzmarkierung von molekularen Substanzen erfolgen. Dazu werden insbesondere fluoreszenzmarkierte Antikörper eingesetzt, die aufgrund ihrer Bindungseigenschaften lange und spezifisch an die vorgegebenen Antigene binden können. Für Rezeptoren bzw. Enzyme ist die Verwendung fluoreszenzmarkierter Liganden, Cofaktoren, Substrate, oder Substratanaloga besonders geeignet. Ein weiterer Anwendungsfall ist die Verwendung interkalierender Substanzen, wie beispielsweise Phenanthridine (Ethidiumbromid), oder Acridine und Cyanine. Die Ausbildung von amyloidogenen Strukturen kann durch die Anwendung des Farbstoffes Congorot und durch nachfolgende Fluoreszenzmessung nachgewiesen werden.

Als wichtige Anwendung für die beschriebene Erfindung kann die Diagnose von amyloidogenen Erkrankungen dienen. Dabei wird biologisches Material aus dem Patienten, beispielsweise aufgeschlossenen Geweben, Liquor, Blut, Urin, oder andere Körperflüssigkeit mit einem oder mehreren verschiedenen fluoreszenzmarkierten Protein(en) oder Peptid(en) (Marker) vermischt und für eine definierte Zeit inkubiert. In dieser Zeit aggregieren bzw. assoziieren die im Patientenmaterial potentiell vorhandenen molekularen Substanzen, entweder in Form von löslichen monomeren Peptiden bzw. Proteinen, oder bereits in Form von Kristallisationskeimen (erste Assoziate) für den Assoziationsprozeß, spezifisch mit dem eingesetzten Marker. Durch geeignete Wahl der Prozeßparameter wie Temperatur, Inkubationsdauer, Lösungsmittelzusätze, pH-Wert, usw. kann nach Optimierungs- und Differenzierungsstrategien, die der Fachmann nach an sich bekannten Strategien durchführt, eine Unterscheidung von monomeren Formen oder auch von bereits gebildeten Assoziaten, oder von Kristallisationskeimen zur Bildung der Assoziate erfolgen. Dabei können zelluläre Faktoren oder Katalysatoren, die in der Testlösung des Patienten mit vorhanden sind, zur spezifischen Bildung mit beitragen, ohne daß die Nachweisgenauigkeit davon beeinträchtigt wird.

In einem Arbeitsgang kann mit dem beschriebenen Verfahren eine Mengenbestimmung für verschiedene molekulare Substanzen durchgeführt werden; dabei wird die relative Häufigkeit des Auftretens von Assoziaten (bei denen verschiedene Typen durch unterschiedliche Fluoreszenzmarkierung voneinander unterscheidbar sind) im Verlauf des Auszählvorganges im FACS-Gerät als proportional zur Menge an Teilstrukturen gewertet, die in der Testlösung (beispielsweise Patientenliquor) vorliegt. Durch Verwendung unterschiedlicher Fluoreszenzfarbstoffe für verschiedene Marker können beispielsweise das Tau-Protein, das Alzheimer-β-Peptid(1-42) und das Alzheimer-β-Peptid(1-40) parallel detektiert werden. Gleichzeitig kann durch den Einsatz von Congorot der amyloidogene Charakter .der entstehenden Aggregate nachgewiesen werden. Moderne FACS-Geräte unterstützen die parallele Detektion von vier Fluoreszenzen und ermöglichen damit eine solche Präzisierung der diagnostischen Aussage. Für das Verfahren wird außerordentlich-wenig Probenmaterial benötigt, da hochempfindliche Einzelpartikelmessungen durchgeführt werden, dies ist von Vorteil für den Patienten. Die Vielzahl an vorstehend beschriebenen möglichen Meßparametern kann eine präzise Kategorisierung der Eigenschaften der Patientenprobe und damit in diagnostischer Hinsicht eine gute quantitative Bestimmung des Krankheitsbildes bzw. des Verlaufes und des Krankheitsfortschritts, nach entsprechender Standardisierung, erlauben.

Neben den diagnostischen Anwendungen im Bereich der amyloidogenen Erkrankungen ist auch das Screening von potentiellen therapeutischen Substanzen mit dem beschriebenen Verfahren denkbar. Als Meßgröße dient hier die Bildung amyloidogener Aggregate in Gegenwart des Therapeutikums; es ist zu erwarten, daß Substanzen, die die Ausbildung der amyloidogenen Aggregate verhindern, auch therapeutisch wertvoll sein können. Ebenso können Substanzen, die die genannten amyloidogenen Aggregate wieder auflösen können, von therapeutischem Nutzen sein.

Neben der Verwendung von Markern, die (mit Ausnahme der Ftuoreszenzmarkierung) identisch mit der zu detektierenden Substanz sind, kann auch die Verwendung von Markern vorteilhaft sein, die nur teilweise mit der zu detektierenden Substanz identisch sind. Hier können insbesondere Proteine. Proteinfragmente, oder Peptide eingesetzt werden, die weitgehend homolog zur Zielsubstanz sind, aber an einer oder an mehreren Stellen Austausche in der Aminosäuresequenz besitzen. Solche homologen Sequenzen können insofern vorteilhaft für das Verfahren sein, als sie andere und damit oft günstigere Assoziations- und Anlagerungseigenschaften besitzen können als die natürlichen Sequenzen. Die Bestimmung solcher homologer Sequenzen kann durch die geeignete Verwendung des in der vorliegenden Erfindung beschriebenen Verfahrens auf einfache Weise erfolgen. Weiterhin kann die Kinetik der Bildung der Aggregate und Assoziate mit dem Verfahren einfach ermittelt werden, und dadurch die diagnostische und analytische Aussage des jeweiligen Verfahrens nochmals erweitert werden. So können insbesondere bei der Diagnostik amyloidogener Erkrankungen verschiedene kinetische Phasen unterschieden werden. Eine schnelle Zusammenlagerung weiterer Moleküle zu größeren Aggregaten oder Assoziaten erfolgt oft erst nach der initialen (und langsamen) Bildung eines Kristallisationskeimes. Die Zugabe eines künstlichen Aggregationskeimes kann somit die zu beobachtenden Prozesse von Aggregation oder Assoziation beschleunigen.

Neben den in FACS-Geräten standardmäßig vorhandenen optischen und spektroskopischen Möglichkeiten können die Molekülassoziate nach geringfügiger technischer Erweiterung der Geräte auch gemäß weiterer Eigenschaften nach an sich bekannten Trennverfahren voneinander separiert werden, wobei die getrennten molekularen Assoziate aufgrund ihrer optischen oder andere Eigenschaften wie beispielsweise Absorptionseigenschaften, circulardichroitische oder lineardichroitische Eigenschaften, Quantenausbeute, Lebensdauer angeregter Zustände, Energietransfer, Intensitätsunterschiede, oder Radioaktivität charakterisierbar sind.

Das in der vorliegenden Erfindung beschriebene Verfahren erlaubt die Charakterisierung von molekularen Assoziaten, wie sie in Anspruch 1 definiert sind, mit beliebigen Mischungsverhältnissen mit Hilfe von fluoreszierenden Markierungen. Die Charakterisierung erfolgt dabei durch statistische Auswertung von Ensembles von Assoziaten oder Aggregaten, wobei als Besonderheit jedes einzelne Assoziat oder Aggregat (Partikel) vermessen werden kann. Als weitere Besonderheit können die molekularen Partikel parallel zur Charakterisierung auch bezüglich wesentlicher Eigenschaften sortiert und gezählt werden. So ist nachfolgend zur FACS-Analyse die Untersuchung der gesammelten Spezies mit Hilfe anderer analytischer Methoden, wie beispielsweise Elektronenmikroskopie, Fluoreszenzmikroskopie, Fluoreszenz-Korrelations-Spektroskopie, etc. möglich.

Die durch FACS charakterisierten und sortierten molekularen Assoziate, die _bestimmte Eigenschaften aufweisen, können weiterhin in nachfolgenden Experimenten (beispielsweise in Zellkulturen, Tiermodellen, oder anderen Untersuchungen mit Bedarf an qualitativ hochwertigem und homogenem Ausgangsmaterial) Anwendung finden. Dadurch ist es möglich, hinsichtlich gewünschter Eigenschaften genau definierte und charakterisierte molekulare Partikel einer homogenen Population einzusetzen. Dies kann in vielen Fällen wesentlich für das Experiment sein.

Ein wesentlicher Vorteil des beschriebenen Verfahrens ist die Standardisierung und die zweite Verbreitetung des Verfahrens FACS. Diese Technik ist an allen diagnostischen Zentren etabliert; medizinische Testverfahren, die darauf beruhen, haben dadurch einen erheblichen Infrastrukturvorteil. FACS wird generell im Durchfluß betrieben, das heißt, die zu vermessende Probe läuft kontinuierlich durch das Gerät. Ein solches Durchflußsystem ist besonders gut geeignet für eine Automatisierung im Hinblick auch auf ein Hochdurchsatzverfahren (High-Throughput-Screening). Nach jedem Probendurchgang kann das Gerät automatisch kurz gespült werden, es sind daher keine manuellen Arbeiten oder der Austausch von Einweg-Material notwendig. Durch die Verwendung eines automatischen Probenauftragungsgerätes kann damit an einem Arbeitsplatz ohne wesentlichen Aufwand seitens des Bedienungspersonals ein großer Probendurchsatz erreicht werden.

Das Verfahren erlaubt es, für jedes der Partikel die mengenmäßige Zusammensetzung aus verschiedenen Teilstrukturen zu bestimmen, sofern diese verschiedenen Teilstrukturen mit unterschiedlichen Fluoreszenzfarbstoffen spezifisch markiert werden können. Dadurch kann eine sehr genaue Quantifizierung durchgeführt werden, die neben Mittelwerten für die Zusammensetzung auch präzise Aussagen über die statistische Verteilung der Zusammensetzung innerhalb der Population liefert. Die Partikel müssen darüber hinaus nicht notwendigerweise direkt markiert werden; sofern beispielsweise spezifische Liganden oder-Antikörper gegen die zu bestimmenden Teilstrukturen zur Verfügung stehen, kann auch eine indirekte Markierung mit Fluoreszenzfarbstoffen erfolgen.

Schließlich kann die molekulare Zusammensetzung der Partikel analysiert werden, beispielsweise bezüglich der Verpackungseffizienz von virusanalogen Proteinhüllen hinsichtlich therapeutisch wirksamer Substanzen wie DNA, RNA, Peptiden, oder Proteinen. Derartige Anwendungen sind beispielsweise im Bereich der Herstellung gentherapeutischer Vektorsysteme relevant. Damit ist ein präzises analytisches Werkzeug gegeben, um solche Systeme zu charakterisieren und nachfolgend zu optimieren.

Ausführungsformen dieser Erfindung sind in den folgenden Beispielen beschrieben, die den Umfang der Erfindung jedoch nicht beschränken sollen. In den Beispielen und in der Beschreibung wird auf die folgenden Figuren Bezug genommen.

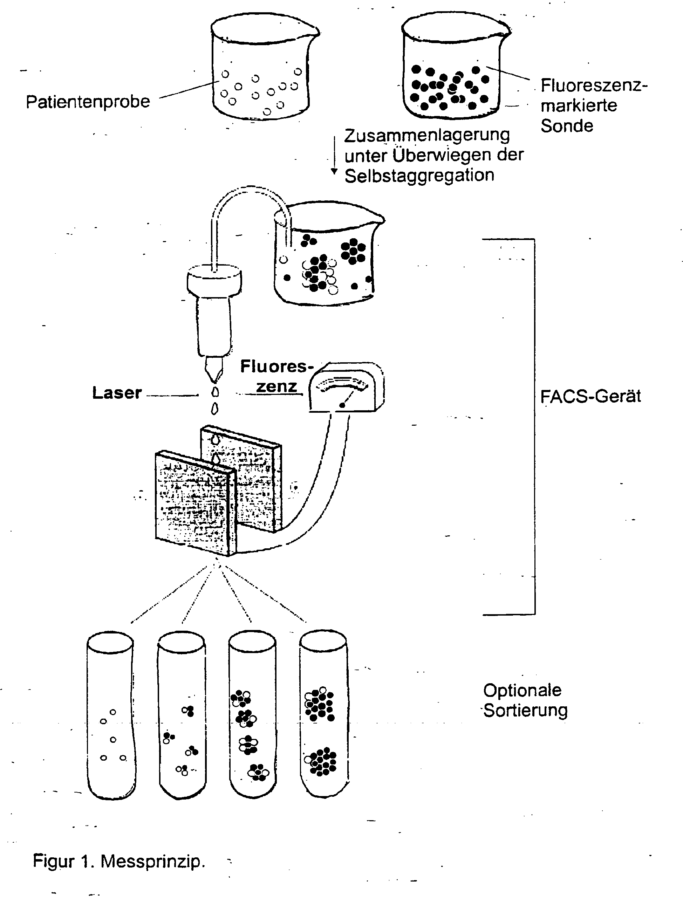

Figur 1 zeigt schematisch ein Beispiel zur Charakterisierung einer Probe mittels FACS. In diesem Beispiel werden fluoreszenzmarkierte Teileinheiten mit unmarkierten Teileinheiten und/oder Assoziaten einer bestimmten Spezies zu markierten molekularen Assoziaten zusammengelagert. Nach Anregung mittels Laser werden die Größen- und Fluoreszenzsignale gemessen, wonach eine bestimmte, vorher definierte Auftrennung und Sortierung gemäß dem Funktionsprinzip von FACS-Geräten (in diesem Falle hinsichtlich der Größe von Aggregaten oder Assoziaten) möglich ist.

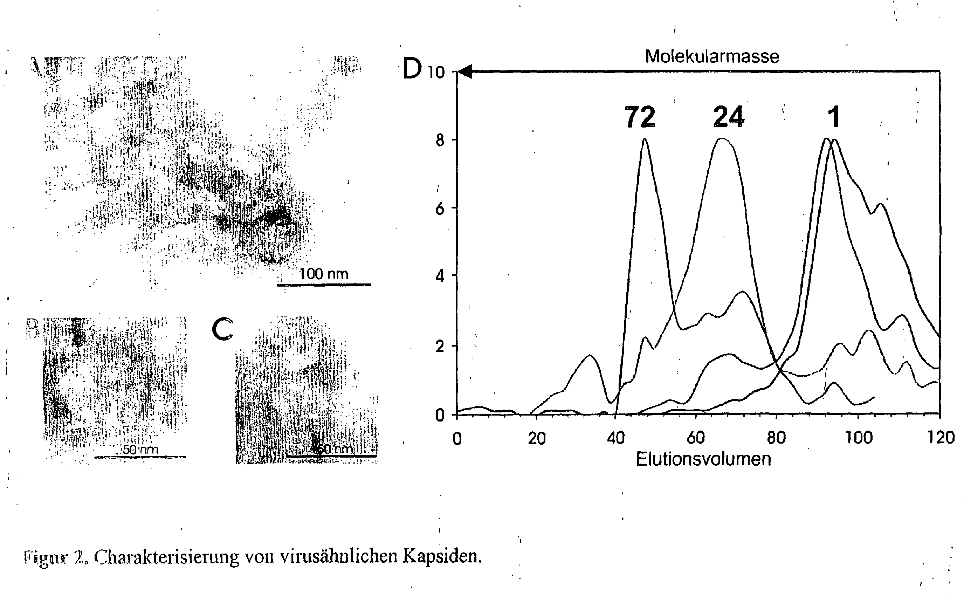

Figur 2 zeigt elektronenmikroskopische Aufnahmen und Gelfiltrationsuntersuchungen von mit Hilfe der FACS-Technologie charakterisierten virusähnlichen Kapsiden (A), nichtassemblierte Kapsomere, abgeleitet von Polyomavirus VP1 (B), 45-nm-Partikel von virusähnlichen Polyomavirus-Hüllen nach Dialyse bei pH 7.2. (C), 30-nm-Partikel nach Dialyse bei pH 8.5. (D), Gelfiltrationsuntersuchungen zur Größenverteilung der virusähnlichen PolyomaVirus-Hüllen.

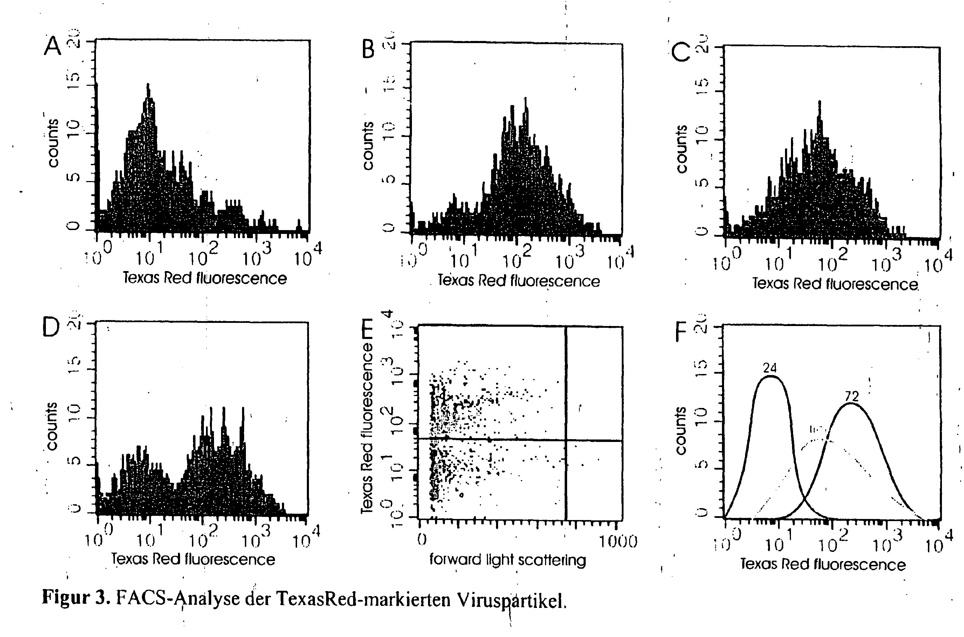

Figur 3 zeigt die zu Figur 2 zugehörige FACS-Analyse von TexasRed-markierten virusähnlichen Polyomavirus-Partikeln. A, Analyse der Partikel, bestehend aus 24 Kapsomeren. B, Analyse der Zusammenlagerung von 72-Pentamer-Kapsiden. C, Assemblierungsdefiziente Variante PyVP1-ΔCT63. D, Kapsomere unter nicht-Assembilerungsbedingungen (keine virusähnlichen Polyomavirus-Partikel). E. Dot-plot der Kapsomere unter nicht-Assembliemngsbedingungen. Die Vorwärts-Lichtstreuung ist aufgrund der extrem kleinen_ Partikelgrößen (5 nm) sehr gering F, Schematische Histogrammdarstellung der fluoreszierenden virusähnlichen Partikel bzw. freien Pentamere verschiedener Größe.

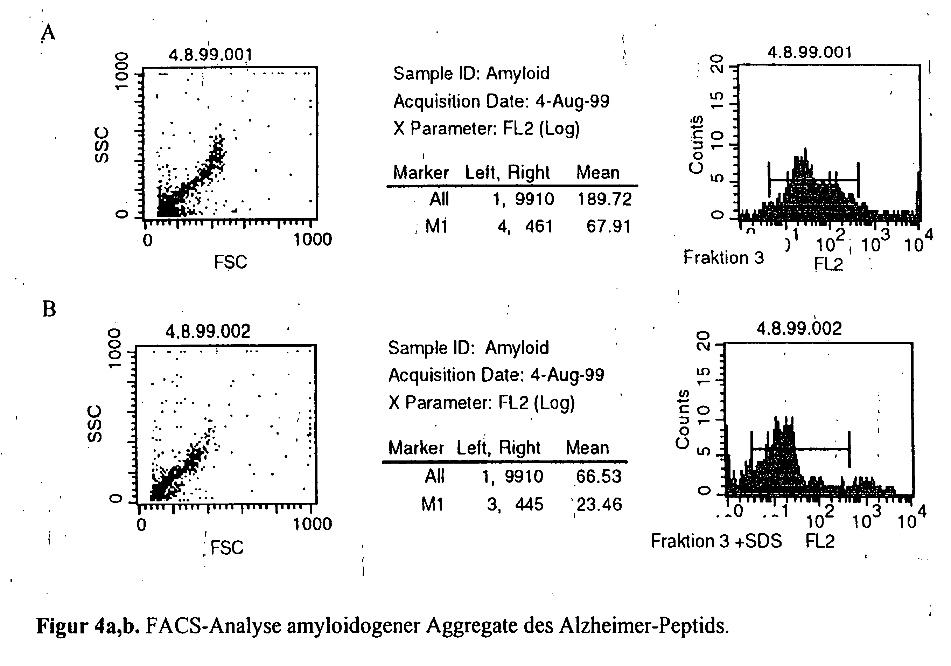

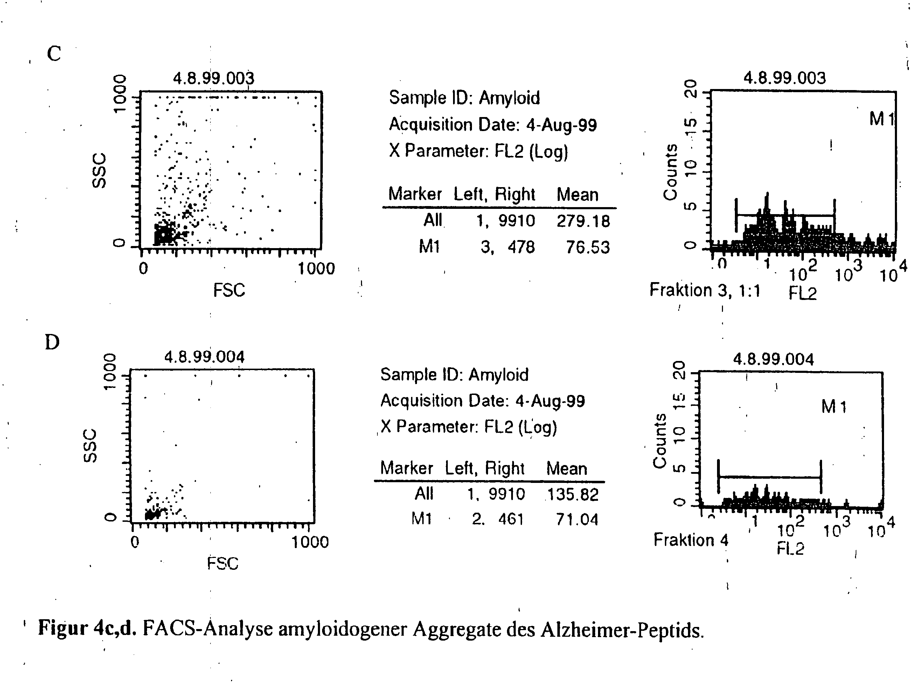

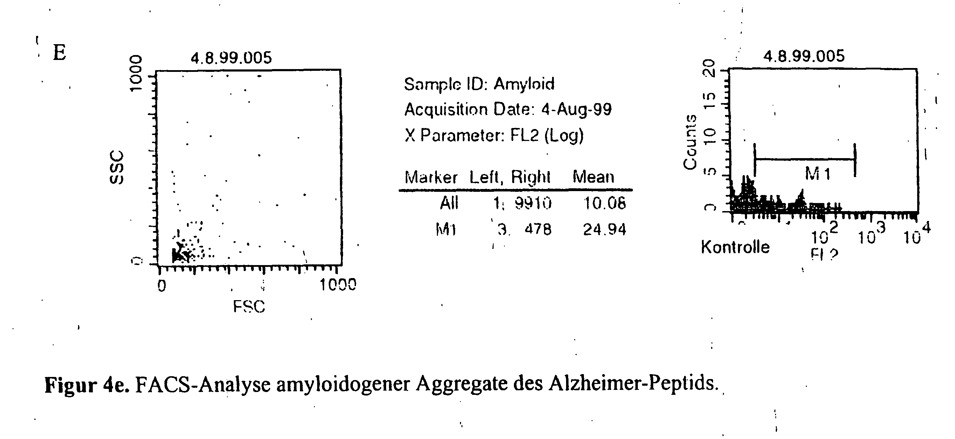

Figur 4 zeigt die Ergebnisse einer FACS-Analyse von Alzheimer-β-Peptid(1-42) unter verschiedenen Aggregationsbedingungen. A, fluoreszenzmarkierte Aggregate bei 0.1 % SDS-Gehalt der Lösung. B, bei einem SDS-Gehalt von 2 % im Lösungsmittel treten die Aggregate unter ansonsten identischen Bedingungen nicht auf; C, eine Mischung von fluoreszenzmarkierten und nichtmarkierten Peptiden bildet unter den Bedingungen von (A) Aggregate mit geringerer Fluoreszenz; D, Kontrollexperiment unter Verwendung von Fluoreszenz-Farbstoff ohne Peptid (Negativkontrolle); E, Kontrollexperiment unter identischen Bedingungen, jedoch unter Verwendung von Alzheimer-β-Peptid(1-42), das zuvor nicht fluoreszenzmarkiert wurde (Negativkontrolle).

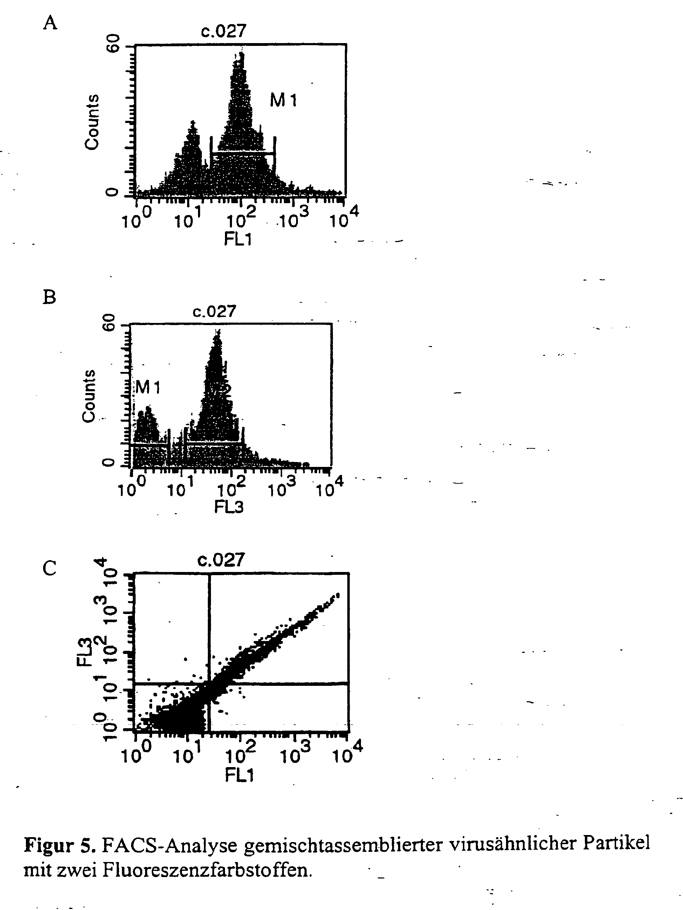

Figur 5 zeigt eine FACS-Analyse unterschiedlich markierter PyVP1-Varianten. Es werden Kapside aus PyVP1-CallS-T249C gebildet, die aus einer mit Fluorescein markierten und einer mit Texas Red markierten Spezies bestehen. Die Kapsidpopulation zeigt eine deutliche Fluorescein-Fluoreszenz (M1 in A), sowie auch eine Texas Red-Fluoreszenz (M2 in B). Aus der Auftragung Fluorescein- (FL1) gegen Texas Red-(FL3) Fluoreszenz wird ersichtlich, daß beide Farbstoffe auf einem Partikel lokalisiert sind (Quadrant oben rechts in C). Partikel, die nur einen Farbstoff enthalten, werden nicht detektiert

### Beispiel 1

### Herstellung fluöreszenzmarkierter Virushüllen definierter Größe, und Charakterisierung der Virushüllen mittels FACS

Das im gegebenen Beispiel verwendete virale Hüllprotein ist das in Lösung pentamere Polyomavirus-VP 1-Protein, das nach dem Stand der Technik einfach *in vitro* zu einer Hülle assemblierbar ist. Dazu wird im ersten Schritt eine Polyomavirus-Variante hergestellt, die keinerlei Cysteine in der Sequenz aufweist; die sechs Cysteine des Wildtyp-Proteins (Cys-12, Cys-16, Cys-20, Cys-115, Cys-274, und Cys-283) werden mit Hilfe von Mutagenisierungsverfahren nach dem Stand der Technik durch Serine ersetzt. Das hat unter anderem den Vorteil, daß die Redoxbedingungen der Lösung keinen Einfluß auf den Zustand des Proteins haben; es ist dadurch in vielen Anwendungen einfacher handhabbar.

Die Mutagenese wird mit Hilfe des QuickChange-Verfahrens (Fa. Stratagene), nach Angaben des Herstellers, durchgeführt. Zur Mutagenese werden die folgenden Oligonukleotide verwendet: C12S, C16S, C20S: 5'-GTC TCT AAA AGC GAG ACA AAA AGC ACA AAG GCT AGC CCA AGA CCC-3', und 5'-GGG TCT TGG GCT AGC CTT TGT GCT TTT TGT CTC GCT TTT AGA GAC-3', C115S: 5'-GAG GAC CTC ACG TCT GAC ACC CTA C-3' und 5'-GTA GGG TGT CAG ACG TGA GGT CCT C-3'; C274S, C283S: 5'-GGG CCC CTC AGC AAA GGA GAA GGT CTA TAC CTC TCG AGC GTA GAT ATA ATG-3' und 5'-CAT TAT ATC TAC GCT CGA GAG GTA TAG ACC TTC TCC TTT GCT GAG GGG CCC-3'.

Dazu kann ein weiteres Protein hergestellt werden, das am C-Terminus um 63 Aminosäuren verkürzt ist. Der C-Terminus ist für die Assemblierung essentiell, die beschriebene Variante des Hüllproteins ist daher assemblierungsdefizient. Die Herstellung der verkürzten Variante PyVP1-ΔCT63 erfolgt mit Hilfe des Oligonukleotids 5'-ATT ACC CGG GAT AGG GAT TTT TGA CCC ATC-3'.

Zur spezifischen Markierung der Kapsomere kann ein singuläres Cystein in eine spezielle Region im Protein eingeführt werden. Dies ist beispielsweise die Position 249, wo ein Threonin gegen ein Cystein ausgetauscht wird. Die Mutagenese erfolgt mit Hilfe des QuickChange-Verfahrens (Fa. Stratagene) nach Herstellerangaben, unter Verwendung der Oligonukleotide 5'-GGA CGG GTG GGG TGC ACG TGC GTG CAG TG-3' und 5'-CAC TGG AGG CAC GTG CAC CCC ACC CGT CC -3'.

Die Assemblierung des nach Standardverfahren hergestellten Proteins PyVP1 -Calls-T249C erfolgt zunächst in Analogie zu bereits vorbeschriebenen Bedingungen nach dem Stand der Technik (vgl. Salunke, Caspar & Garcea, Biophys. J. 56, S. 887-900, 1989). Dabei kommen zwei Assemblierungsvarianten zum Einsatz. Die virusähnlichen Kapside mit Durchmesser 45 nm (bestehend aus 72 Kapsomeren) werden nach Dialyse des Proteins gegen 10 mM HEPES, 50 mM NaCl, 0.5 mM CaCl₂. 5 % Glycerin, pH 7.2, für 72 Stunden bei Raumtemperatur erhalten. Dagegen entstehen sehr viel kleinere Partikel (Durchmesser 30 nm), bestehend aus 24 Kapsomeren, durch die Dialyse gegen 10 mM HEPES, 50 mM NaCl, 0.5 mM CaCl₂, 5 % Glycerin, pH 8.5. für 72 Stunden bei Raumtemperatur.

Das PyVP1-CallS-T249C-Protein wird im Verfahren als lösliches Pentamer exprimiert und ist nativ, das heißt, assemblierungskompetent. In Fig. 2 ist ein Gelfiltrationsexperiment dargestellt, das zeigt, daß das PyVP1-CallS-T249C-Protein unter geeigneten Bedingungen zu kapsidähnlichen Strukturen verschiedener Größe assembliert werden kann. Fig. 2 beschreibt ebenfalls die entstandenen Kapside mit Hilfe von elektronenmikroskopischen Aufnahmen.

Das gereinigte Kapsomer kann vor der Assemblierung mit den Farbstoffen Fluorescein-Maleimid oder Texas Red-Maleimid (Fa. Molecular Probes) nach Herstellerangaben markiert werden. Dabei erfolgt eine spezifische Kopplung an der Stelle des singulären Cysteins 249.

Die Figur 3 stellt das Ergebnis einer FACS-Analyse der assemblierten Partikel dar. Die Partikel sind überraschenderweise gut im FACS detektierbar und können auch voneinander unterschieden werden, so daß eine Analyse der Größe und der Verteilung der Zusammensetzung für jedes einzelne Partikel erfolgen kann.

### Beispiel 2

### Charakterisierung der Aggregation des Alzheimer-β-Peptids

Das Alzheimer-β-Peptid(1-42) wird in synthetischer Form kommerziell von der Firma Sigma vertrieben. Das Peptid wird in Puffer gelöst, der 10 mM HEPES, 50 mM NaCl, und 2 % SDS, pH 7.2, enthält. Das Peptid kann mit Hilfe des Farbstoffes Rhodamin-X-succinimidylester (Fa. Molecular Probes) nach Angaben des Herstellers über Aminogruppen fluoreszenzmarkiert werden. Der überschüssige Farbstoff kann anschließend durch eine Gelfiltrationssäule vom Peptid weitgehend abgetrennt werden. Das so markierte Peptid (aus Fraktion 3 der Gelfiltration) wird in drei parallelen Experimenten eingesetzt. Zum einen wird es durch Verdünnen (1:20) mit SDS-freiem Puffer zur Aggregation veranlaßt. Die entstehenden Aggregate können mit Hilfe des FACS-Verfahrens nachgewiesen werden (Fig. 4A). Wird zur Kontrolle (Experiment 2) mit SDS-haltigem Puffer (2 % SDS, w/v) verdünnt, so entstehen keine. Aggregate und die spezifischen FACS-Signale an dieser Stelle unterbleiben (Fig. 4B).

Wird im dritten Experiment SDS-freier Puffer zugesetzt, der zusätzlich unmarkiertes Alzheimer-β-Peptid enthält; dann treten wie erwartet wiederum Aggregate auf, die jedoch ein geringeres Fluoreszenzsignal zeigen: es wurde offenbar in die entstehenden Aggregate (die laut der Streukurven eine ähnliche -Größenverteilung zeigen) nichtmarkiertes Peptid mit eingebaut (Fig. 4C).

Zur Kontrolle wurde auch der gelöste Fluoreszenzfarbstoff (Fraktion 4 der Gelfiltration) vermessen (Fig. 4D), der kein spezifisches FACS-Signal an der Stelle der Alzheimer-β-Peptid(1-42)-Aggregate liefert. Ebenfalls zeigt das unmarkierte Peptid als Kontrolle (Fig. 4E) kein spezifisches FACS-Signal.

Das Beispiel demonstriert, daß mit Hilfe der FACS-Technologie Größe, Art und Zusammensetzung von Aggregaten, bestehend beispielsweise aus dem Alzheimer-β-Peptid, spezifisch charakterisiert werden können. Gleichzeitig ist gezeigt, daß nichtmarkiertes Peptid der gleichen chemischen Natur, wie es beispielsweise im Patientenliquor auftreten kann, in die amyloidogenen Aggregate mit eingebaut werden kann. Mit diesem Verfahren ist damit die Möglichkeit geschaffen, hoch sensitiv und spezifisch molekulare Assoziate und Aggregate zu charakterisieren, die pathologischen Ablagerungen entsprechen.

### Beispiel 3

### Herstellung und Charakterisierung von gemischten Kapsiden

Die Charakterisierung von gemischten Proteinhüllen (Kapsiden), das heißt, Partikel, die mosaikartig aus mehreren verschiedenen molekularen Substanzen aufgebaut sind, ist eine besonders elegante Nachweismöglichkeit der vorliegenden Erfindung. Zum Nachweis gemischter, aus unterschiedlichen Hüllproteinen aufgebauter Kapside, wird die Variante PyVPI-CallS-T249C des Hüllproteins (siehe Beispiel 1) in einem Ansatz mit dem Fluoreszenzfarbstoff Fluorescein-Maleimid und in einem zweiten Ansatz mit Texas Red-Maleimid an das singuläre Cystein 249 gekoppelt. Die verschieden markierten Kapside werden in einem äquimolaren Verhältnis miteinander gemischt und assembliert. Die Analyse der Kapsidbildung erfolgt mittels FACS. Diese ermöglicht eine Detektion unterschiedlicher Fluoreszenzen innerhalb eines Partikels. Figur 5 zeigt die Analyse äquimolar assemblierter Kapside. Es zeigt sich jeweils eine Population fluoreszenzmarkierter Kapside und freier nichtassemblierter Kapsomere (Figur 5A, 5B). Bei der Auftragung Fluorescein- gegen Texas Red-Fluoreszenz (Figur 5C) wird eine Population von Partikeln beobachtet, die gleichzeitig beide Fluoreszenzen tragen. Partikel, die nur mit einem Farbstoff markiert sind, sind dagegen nicht vorhanden.

Mit diesem Beispiel wird gezeigt, daß sich mosaikartig assemblierte Polyomavirus VPI-Hüllproteine mit dem beschriebenen Verfahren charakterisieren lassen, und es kann nachgewiesen werden, daß jedes einzelne Partikel, das im Verlauf der Assemblierung entsteht, beide verschieden fluoreszenzmarkierte Kapsomertypen eingebaut hat. Im Gegensatz zu allen anderen spektroskopischen Verfahren, die eine Mittelung aller im Lichtstrahl befindlichen Fluoreszenzen messen, erlaubt das Verfahren die Bestimmung der Verteilung der Zusammensetzung von molekularen Assoziaten und Aggregaten auf der Basis vieler einzelner Partikel. Dadurch kann neben der Charakterisierung einzelner Partikel auch die statistische Verteilung der in je ein Partikel eingebauten Teilstrukturen bestimmt werden, sofern diese mit unterschiedlichen Fluoreszenzfarbstoffen markiert wurden.

## Patentansprüche

1. Verfahren zur Charakterisierung von aus Teileinheiten bestehenden molekularen Assoziaten, wobei:
- nicht assoziierte Teileinheiten mit mindestens einem fluoreszierenden Farbstoff markiert werden,
- die markierten Teileinheiten untereinander oder mit nicht markierten Teileinheiten oder mit aus Teileinheiten bestehenden molekularen Assoziaten in Kontakt gebracht werden, um eine Anlagerung und/oder Bindung der markierten Teileinheiten aneinander oder an nicht markierte Teileinheiten oder an die aus Teileinheiten bestehenden molekularen Assoziate zu erreichen, um markierte molekulare Assoziate zu bilden,
und
- die markierten molekularen Assoziate mittels eines FACS (Fluorescence-Activated Cell Sorter) charakterisisert werden, die zu charakterisierenden molekularen Assoziate einen Größenbereich von 1 bis 1000 nm aufweisen, und
- die molekularen Assoziate Viren- oder Phagenkapside, Proteasomen, Chaperon-komplexe oder Ribososomen sind oder aus modifizierten Teileinheiten hiervon zusammengesetzt sind, oder aus gleichen Teileinheiten bestehende Peptid- oder Protein-Assoziate, einzelsträngige oder doppelsträngige DNA, einzelsträngige oder doppelsträngige RNA, Lipid-Assoziate, Phospholipid-Assoziate, Kohlenhydrat-Assoziate, Polysaccharid-Assoziate, Assoziate enthaltend Kohlenwasserstoff-Verbindungen mit hydrophilem oder hydrophobem Charakter, Assoziate aus isoprenoiden Verbindungen, oder Gemische hiervon sind.

2. Verfahren nach Anspruch 1,
**dadurch gekennzeichnet,**
**daß** die Teileinheiten Monomere, Dimere und/oder Oligomere sein können.

3. Verfahren nach Anspruch 1 oder 2,
**dadurch gekennzeichnet,**
**daß** die Teileinheiten der molekularen Assoziate gleich oder voneinander verschieden sein können.

4. Verfahren nach einem oder mehreren der vorhergehenden Ansprüche,
**dadurch gekennzeichnet,**
**daß** die molekularen Assoziate nach an sich bekannten Verfahren getrennt werden.

5. Verfahren nach einem oder mehreren der vorhergehenden Ansprüche,
**dadurch gekennzeichnet,**
**daß** die molekularen Assoziate Virus- oder Phagenkapside aus der Gruppe der Adenoviridae, Arenaviridae, Arterivirus, Astroviridae, Bacilloviridae, Baculoviridae, Badnavirus, Barnaviridae, Birnaviridae, Bromoviridae, Bunyaviridae, Caliciviridae, Capillovirus, Carlavirus, Caulimovirus, Circoviridae, Closterovirus, Comoviridae, Coronaviridae, Corticoviridae, Cystoviridae, Dianthovirus, Enamovirus, Filoviridae, Flaviviridae, Furovirus, Fuselloviridae, Geminiviridae, Guttaviridae, Hepadnaviridae, Herpesviridae, Hordeivirus, Hypoviridae, Idaeovirus, Inoviridae, Iridoviridae, Leviviridae, Lipothrixviridae, Luteovirus, Machlomovirus, Marafivirus, Microviridae, Myoviridae, Necrovirus, Nodaviridae, Orthomyxoviridae, Papovaviridae, Paramyxoviridae, Partitiviridae, Parvoviridae, Phycodnaviridae, Picornaviridae, Plasmaviridae, Podoviridae, Polydnaviridae, Potexvirus, Potyviridae, Poxviridae, Reoviridae, Retroviridae, Rhabdoviridae, Rhizidiovirus, Sequiviridae, Siphoviridae, Sobemovirus, Tectiviridae, Tenuivirus, Tetraviridae, Tobamovirus, Tobnavirus, Togaviridae, Tombusviridae, Totiviridae, Trichovirus, Tymovirus, Umbravirus sind oder hiervon abgeleitet sind oder aus einer oder mehreren modifizierten Teileinheiten der genannten Viren oder Phagen abgeleitet sind.

6. Verfahren nach einem der Ansprüche 1 bis 4,
**dadurch gekennzeichnet,**
**daß** die Teileinheiten der molekularen Assoziate Alzheimer-β-Peptide, Tau-Proteine, Prion-Proteine, Huntingtin, Synuclein, SCA1 / Ataxin-1, Cystatin C, Immunglobuline. Lipoproteine, Transthyretin, Apolipoprotein A-1, Serum-Amyloid A, Islet-Amyloid-Polypeptid. Insulin, Calcitonin, β-2-Mikroglobulin, Lysozym, Fibrinogen, Gelsolin, Atrial Natriuretic Factor, HoxD13-Genprodukt, Transkriptionsfaktor CBFA1, Poly(A)-Bindungsprotein II, oder modifizierte Formen oder Fragmente hiervon sind.

7. Verfahren nach Anspruch 6,
**dadurch gekennzeichnet,**
**daß** molekulare Assoziate charakterisiert werden, die bei den neurodegenerativen Erkrankungen Morbus Alzheimer, Transmissible Spongiforme Enzephalopathie. Chorea Huntington, Morbus Parkinson, Spinocerebellare Ataxie Typ I, und vererbbare Cerebrale Amyloide Angiopathie oder bei den Erkrankungen Primäre oder Reaktive Asystemische Amyloidose, Sekundäre Systemische Amyloidose, Familiäre Amyloide Polyneuropathie 1 und III, Diabetes mellitus Typ II, Injektionslokalisierte und Dialyseassoziierte Amyloidose, Medullaria-Carcinom der Schilddrüse, β-2-Mikroglobulin-Amyloidose, Nichtneuropathische Amyloidose, Vererbte Renale Amyloidose, Finnische vererbte Systemische Amyloidose, Atriale Amyloidose, Typ II-Syndactylie, Machado-Joseph-Krankheit, Cleidocraniale Dysplasie und Okularpharyngeale Muskeldystrophie auftreten.

8. Verfahren nach einem oder mehreren der vorhergehenden Ansprüche,
**dadurch gekennzeichnet,**
**daß** die zu charakterisierenden Assoziate einen Größenbereich von 10 bis 300 nm, bevorzugt 10 bis 100 nm, weiterhin bevorzugt 1 bis 100 nm, aufweisen.

9. Verwendung des Verfahrens FACS zur Charakterisierung von aus Teileinheiten bestehenden molekularen Assoziaten bezüglich Größe und Zusammensetzung, wobei mindestens eine der Teileinheiten eines Assoziats mit mindestens einem Fluoreszenzfarbstoff markiert ist, wobei die molekularen Assoziate durch ein Verfahren nach einem der Ansprüche 1-8 erhalten wurden.

10. Verwendung nach Anspruch 9 zur Charakterisierung von aus Teileinheiten bestehenden molekularen Assoziaten, die mit den neurodegenerativen Erkrankungen Morbus Alzheimer, Transmissible Spongiforme Enzephalopathie, Chorea Huntington, Morbus Parkinson, Spinocerebellare Ataxie Typ I, und vererbbare Cerebrale Amyloide Angiopathie oder den Erkrankungen Primäre oder Reaktive Asystemische Amyloidose, Sekundäre Systemische Amyloidose, Familiäre Amyloide Polyneuropathie I und III, Diabetes mellitus Typ II, Injektionslokalisierte und Dialyseassoziierte Amyloidose, Medullaria-Carcinom der Schilddrüse, β-2-Mikroglobulin-Amyloidose, Nichtneuropathische Amyloidose, Vererbte Renale Amyloidose, Finnische vererbte Systemische Amyloidose, Atriale Amyloidose, Typ II-Syndactylie, Machado-Joseph-Krankheit, Cleidocraniale Dysplasie und Okularpharyngeale Muskeldystrophie einhergehen.

11. Verwendung nach Anspruch 9,
**dadurch gekennzeichnet,**
**daß** die zu charakterisierenden Assoziate einen Größenbereich von 10 bis 300 nm, bevorzugt 10 bis 100 nm, weiterhin bevorzugt 1 bis 100 nm, aufweisen.

## Claims

1. Method for the characterization of molecular associates consisting of subunits, where:
- non-associated subunits are labeled with at least one fluorescence dye,
- the labeled subunits are brought in contact with each other or with unlabeled subunits or with molecular associates consisting of subunits, in order to reach an association and/or binding of the labeled subunits to each other or to unlabeled subunits or to said molecular associates consisting of subunits, in order to form labeled molecular associates,
and
- the labeled molecular associates are **characterized by** a FACS (Fluorescence-Activated Cell Sorter),
- the molecular associates to be **characterized** have a size range of 1 to 1000 nm, and
- the molecular associates are virus or phage capsids, proteasomes, chaperon complexes, or ribosomes, or are build up from modified subunits thereof, or are peptide associates or protein associates consisting of identical subunits, are single-stranded or double-stranded DNA, are single-stranded or double-stranded RNA, are lipid associates, phospholipid associates, carbohydrate associates, polysaccharide associates, associates containing carbohydrate compounds with hydrophilic or hydrophobic character, associates from isoprenoidic compounds, or mixtures thereof.

2. Method according to claim 1,
**characterized by** the fact,
that the subunits can be monomers, dimers and/or oligomers.

3. Method according to claim 1 or 2,
**characterized by** the fact,
that the subunits of the molecular associates can be identical or can differ from each other.

4. Method according to one or more of the preceding claims,
**characterized by** the fact,
that the molecular associates can be separated by methods known per se.

5. Method according to one or more of the preceding claims,
**characterized by** the fact,
that the molecular associates are virus capsids or phage capsids of the group of Adenoviridae, Arenaviridae, Arterivirus, Astroviridae, Bacilloviridae, Baculoviridae, Batnavirus, Barnaviridae, Birnaviridae, Bromoviridae, Bunyaviridae, Caliciviridae, Capillovirus, Carlavirus, Caulimovirus, Circoviridae, Closterovirus, Comoviridae, Coronaviridae, Corticoviridae, Cystoviridae, Dianthovirus, Enamovirus, Filoviridae, Flaviviridae, Furovirus, Fuselloviridae, Geminiviridae, Guttaviridae, Hepadnaviridae, Herpesviridae, Hordeivirus, Hypoviridae, Idaeovirus, Inoviridae, Iridoviridae, Leviviridae, Lipothrixviridae, Luteovirus, Machlomovirus, Marafivirus, Microviridae, Myoviridae, Necrovirus, Nodaviridae, Orthomyxoviridae, Papovaviridae, Paramyxoviridae, Partitiviridae, Parvoviridae, Phycodnaviridae, Picornaviridae, Plasmaviridae, Podoviridae, Polydnaviridae, Potexvirus, Potyviridae, Poxviridae, Reoviridae, Retroviridae, Rhabdoviridae, Rhizidiovirus, Sequiviridae, Siphoviridae, Sobemovirus, Tectiviridae, Tenuivirus, Tetraviridae, Tobamovirus, Tobravirus, Togaviridae, Tombusviridae, Totiviridae, Trichovirus, Tymovirus, Umbravirus or are derived from the same or are derived from one or more modified subunits of the mentioned viruses or phages.

6. Method according to one of the claims 1 to 4,
**characterized by** the fact,
that the subunits of the molecular associates are Alzheimer-β-peptides, tau proteins, prion proteins, huntingtin, synuclein, SCA1 / ataxin I, cystatin C, immunoglobulins, lipoproteins, transthyretin, apoliproprotein A1, serum amyloid A, islet amyloid polypeptide, insulin, calcitonin, β-2-microglobulin, lysozyme, fibrinogen, gelsolin, atrial natriuretic factor, gene product of hoxD13, transcription factor CBFA1, poly(A)-binding protein II, or are modified forms or fragments thereof.

7. Method according to claim 6,
**characterized by** the fact,
that molecular associates are **characterized** which appear during the neurodegenerative diseases Morbus Alzheimer, Transmissible Spongiform Encephalopathy, Chorea Huntington, Morbus Parkinson, Spinocerebellar Ataxia Type 1, and Hereditary Cerebral Amyloid Angiopathy or during the diseases Primary or Reactive Asystemic Amyloidosis, Secondary Systemic Amyloidosis, Familial Amyloid Polyneuropathy I and III, Diabetes Mellitus Type II, Injection-localized and Dialysis-associated Amyloidosis, Medullary Carcinoma of the thyroid, β-2-Microglobulin Amyloidosis, Non-Neuropathic Amyloidosis, Inherited Renal Amyloidosis, Finnish Inherited Systemic Amyloidosis, Atrial Amyloidosis, Syndactuly Type II, Machado-Josephs Disease, Cleidocranialic Dysplasy and Ocular-pharyngitic Myodystrophy.

8. Method according to one or more of the preceding claims,
**characterized by** the fact,
that the associates to be **characterized** show a size range of 10 to 300 nm, preferably 10 to 100 nm, further preferred 1 to 100 nm.

9. Use of the FACS method for the characterization of molecular associates consisting of subunits, with respect to size and composition, whereby at least one of the subunits of an associate is labeled with at least one fluorescence dye, whereby the molecular associates are defined obtained by a method according to anyone of claims 1-8.

10. Use according to claim 9 for the characterization of molecular associates, consisting of subunits, which appear together with the neurodegenerative diseases Morbus Alzheimer, Transmissible Spongiform Encephalopathy, Chorea Huntington, Morbus Parkinson, Spinocerebellar Ataxia Type 1, and Hereditary Cerebral Amyloid Angiopathy or during the diseases Primary or Reactive Asystemic Amyloidosis, Secondary Systemic Amyloidosis, Familial Amyloid Polyneuropathy I and III, Diabetes Mellitus Type II, Injection-localized and Dialysis-associated Amyloidosis, Medullary Carcinoma of the thyroid, β-2-Microglobulin Amyloidosis, Non-Neuropathic Amyloidosis, Inherited Renal Amyloidosis, Finnish Inherited Systemic Amyloidosis, Atrial Amyloidosis, Syndactuly Type II, Machado-Josephs Disease, Cleidocranialic Dysplasy and Ocular-pharyngitic Myodystrophy.

11. Use according to claim 9,
**characterized by** the fact,
that the associates to be **characterized** have a size range from 10 to 300 nm, preferably 10 to 100 nm, further preferred 1 to 100 nm.

## Revendications

1. Procédé de caractérisation d'associations moléculaires constituées d'unités partielles,
- les unités partielles non associées étant marquées avec au moins un colorant fluorescent,
- les unités partielles marquées étant mises en contact les unes avec les autres ou avec des unités partielles non marquées ou avec des associations moléculaires constituées d'unités partielles pour obtenir une fixation et/ou une liaison des unités marquées l'une à l'autre ou à des unités partielles non marquées ou aux associations moléculaires constituées d'unités partielles afin de former des associations moléculaires marquées, et
- les associations moléculaires marquées étant
**caractérisées** au moyen d'un FACS (Fluorescence-Activated Cell Sorter),
- les associations moléculaires à caractériser présentant un ordre de grandeur de 1 à 1000 nm et
- les associations moléculaires étant des capsides de virus ou de phages, des protéosomes, des complexes chaperons ou des ribosomes ou sont constituées d'unités partielles modifiées de ceux-ci ou d'associations de peptides ou de protéines constituées des mêmes unités partielles, d'ADN à brin simple ou double, d'ARN à brin simple ou double, des associations de lipides, des associations de phospholipides, des associations de glucides, des associations de polysaccharides, des associations contenant des composés d'hydrocarbures présentant un caractère hydrophile ou hydrophobe, des associations de composés isoprénoïdes ou des mélanges de ceux-ci.

2. Procédé selon la revendication 1,
**caractérisé en ce**
**que** les unités partielles peuvent être des monomères, des dimères et/ou des oligomères.

3. Procédé selon la revendication 1 ou 2,
**caractérisé en ce**
**que** les unités partielles des associations moléculaires peuvent être identiques ou différentes les unes des autres.

4. Procédé selon l'une quelconque des revendications précédentes,
**caractérisé en**
**que** les associations moléculaires sont séparées selon des procédés connus en tant que tels.

5. Procédé selon l'une quelconque des revendications précédentes,
**caractérisé en ce**
**que** les associations moléculaires sont des capsides de virus ou de phages du groupe des adénovirus, arénavirus, artévirus, astrovirus, bacillovirus, baculovirus, badnavirus, barnavirus, birnavirus, bromovirus, bunyavirus, calcivirus, capillovirus, carlavirus, caulimovirus, circovirus, clostérovirus, cornovirus, coronavirus, corticovirus, cystovirus, dianthovirus, énamovirus, filovirus, flavivirus, furovirus, fusellovirus, géminivirus, guttavirus, hepadnavirus, herpès-virus, hordéivirus, hypovirus, idaeovirus, inovirus, levivirus, lipothrixvirus, lutéovirus, machlomovirus, marafivirus, microvirus, myovirus, nécrovirus, nodavirus, orthomyxovirus, papovavirus, paramyxovirus, partivirus, parvovirus, phycodnavirus, picronavirus, plasmavirus, podovirus, polydnavirus, potexvirus, potyvirus, poxvirus, réovorus, retrovirus, rhabdovirus, rhizidiovirus, séquivirus, siphovirus, sobémovirus, tectivirus, ténuivirus, tétravirus, tobamovirus, tobravirus, togavirus, tombusvirus, totivirus, trichlovirus, tymovirus, umbravirus ou dérivés de ceux-ci ou dérivés d'une ou de plusieurs unités partielles modifiées des virus ou phages cités.

6. Procédé selon l'une quelconque des revendications 1 à 4,
**caractérisé en ce**
**que** les unités partielles des associations moléculaires sont des peptide β de la maladie d'Alzheimer, des protéines Tau, des protéines de prion, de la maladie d'Huntington, la synucléine, la SCA1/ataxine-1, la cystatine C, les immunoglobulines, les lipoprotéines, la transthyrétine, l'apolipoprotéine A-1, l'amyloïde A sérique, le polypeptide amyloïde des îlots, l'insuline, la calcitonine, la β-2-microglubuline, la lysozyme, le fibrinogène, la gelsoline, le facteur natriurétique auriculaire, le produit de gène HoxD13, le facteur de transcription CBFA1, la protéine de liaison poly(A) II ou des formes modifiées ou des fragments de ceux-ci.

7. Procédé selon la revendication 6,
**caractérisé en ce**
**que** des associations moléculaires sont **caractérisées**, qui surviennent dans les maladies neurodégénératives telles que maladie d'Alzheimer, encéphaline spongiforme transmissible, Chorée de Huntington, maladie de Parkinson, ataxie spinocérébelleuse de type I et angiopathie amyloïde cérébrale héréditaire ou dans les maladies telles que amyloïdose primaire ou asystémique réactive, amyloïdose systémique secondaire, polyneuropathie amyloïde familiale I et III, diabète sucré de type II, amyloïdose localisée à la zone d'injection et associée à la dialyse, carcinome médullaire de la glande thyroïde, amyloïdose β-2-microglobuline, amyloïdose non neuropathique, amyloïdose rénale héréditaire, amyloïdose A systémique héréditaire finlandaise, amyloïdose auriculaire, syndactylie de type II, maladie de Machado-Joseph, dysplasie cléidocrâniale et myodystrophie oculo-pharyngée.

8. Procédé selon une ou plusieurs des revendications précédentes,
**caractérisé en ce**
**que** les associations à caractériser présentent un ordre de grandeur de 10 à 300 nm, de préférence de 10 à 100 nm, de manière davantage préférée, de 1 à 100 nm.

9. Utilisation du procédé FACS pour la caractérisation d'associations moléculaires constituées d'unités partielles eu égard à leur taille et composition, au moins une des unités partielles d'une association étant marquée avec au moins un colorant fluorescent, les associations moléculaires étant obtenues par un procédé selon l'une quelconque des revendications 1 à 8.

10. Utilisation selon la revendication 9 pour la caractérisation des associations moléculaires constituées d'unités partielles qui s'accompagnent de maladies neurodégénératives telles que maladie d'Alzheimer, encéphaline spongiforme transmissible, Chorée de Huntington, maladie de Parkinson, ataxie spinocérébelleuse de type I et angiopathie amyloïde cérébrale héréditaire ou des maladies telles que amyloïdose primaire ou asystémique réactive, amyloïdose systémique secondaire, polyneuropathie amyloïde familiale I et III, diabète sucré de type II, amyloïdose localisée à la zone d'injection et associée à la dialyse, carcinome médullaire de la glande thyroïde, amyloïdose β-2-microglobuline, amyloïdose non neuropathique, amyloïdose rénale héréditaire, amyloïdose A systémique héréditaire finlandaise, amyloïdose auriculaire, syndactylie de type II, maladie de Machado-Joseph, dysplasie cléidocrâniale et myodystrophie oculo-pharyngée.

11. Utilisation selon la revendication 9,
**caractérisée en ce**
**que** les associations à caractériser présentent un ordre de grandeur de 10 à 300 nm, de préférence de 10 à 100 nm, de manière davantage préférée, de 1 à 100 nm.


### SEQUENZPROTOKOLL

<110> ACGT ProGenomics AG
<120> verfahren zur Charakterisierung und zur Auftrennung von molekularen Assoziaten
<130> P12606
<140>
   <141>
<150> DE - 199 52 955.8
   <151> 1999-11-03
<160> 5
<170> Patent In Ver. 2.1
<210> 1
   <211> 1152
   <212> DNA
   <213> Künstliche Sequenz
<220>
   <223> Beschreibung der künstlichen Sequenz: Variante von Polyomavirus VP1 mit allen natürlichen Cysteinen ersetzt durch Serin, und Einführen eines neuen Cysteins anstelle von Thr 249 (PyVP1-CallS-T249C)
<220>
   <221> CDS
   <222> (1)..(1152)
<400> 1
   atg gcc ccc aaa aga aaa agc ggc gtc tct aaa agc gag aca aaa agc     48
   Met Ala Pro Lys Arg Lys Ser Gly Val Ser Lys Ser Glu Thr Lys Ser
   1               5                   10                  15
   aca aag gct agc cca aga ccc gca ccc gtt ccc aaa ctg ctt att aaa     96
   Thr Lys Ala Ser Pro Arg Pro Ala Pro Val Pro Lys Leu Leu Ile Lys
               20                  25                  30
   ggg ggt atg gag gtg ctg gac ctt gtg aca ggg cca gac agt gtg aca     144
   Gly Gly Met Glu Val Leu Asp Leu Val Thr Gly Pro Asp Ser Val Thr
           35                  40                  45
   gaa ata gaa gct ttt ctg aac ccc aga atg ggg cag cca ccc acc cct    192
   Glu Ile Glu Ala Phe Leu Asn Pro Arg Met Gly Gln Pro Pro Thr Pro
           50                  55                  60
   gaa agc cta aca gag gga ggg caa tac tat ggt tgg agc aga ggg att    240
   Glu Ser Leu Thr Glu Gly Gly Gln Tyr Tyr Gly Trp Ser Arg Gly Ile
    65                  70                  75                  80
   aat ttg gct aca tca gat aca gag gat tcc cca gga aat aat aca ctt    288
   Asn Leu Ala Thr Ser Asp Thr Glu Asp Ser Pro Gly Asn Asn Thr Leu
                    85                  90                  95
   ccc aca tgg agt atg gca aag ctc cag ctt ccc atg ctc aat gag gac    336
   Pro Thr Trp Ser Met Ala Lys Leu Gln Leu Pro Met Leu Asn Glu Asp
               100                 105                 110
   ctc acg tct gac acc cta caa atg tgg gag gca gtc tca gtg aaa acc    384
   Leu Thr Ser Asp Thr Leu Gln Met Trp Glu Ala Val Ser Val Lys Thr
           115                 120                 125
   gag gtg gtg ggc tct ggc tca ctg tta gat gtg cat ggg ttc aac aaa    432
   Glu Val Val Gly Ser Gly Ser Leu Leu Asp Val His Gly Phe Asn Lys
       130                 135                 140
   ccc aca gat aca gta aac aca aaa gga att tcc act cca gtg gaa ggc    480
   Pro Thr Asp Thr Val Asn Thr Lys Gly Ile Ser Thr Pro Val Glu Gly
   145                 150                 155                 160
   agc caa tat cat gtg ttt gct gtg ggc ggg gaa ccg ctt gac ctc cag    528
   Ser Gln Tyr His Val Phe Ala Val Gly Gly Glu Pro Leu Asp Leu Gln
                   165                 170                 175
   gga ctt gtg aca gat gcc aga aca aaa tac aag gaa gaa ggg gta gta    576
   Gly Leu Val Thr Asp Ala Arg Thr Lys Tyr Lys Glu Glu Gly Val Val
               180                 185                 190
   aca atc aaa aca atc aca aag aag gac atg gtc aac aaa gac caa gtc    624
   Thr Ile Lys Thr Ile Thr Lys Lys Asp Met Val Asn Lys Asp Gln Val
           195                 200                 205
   ctg aat cca att agc aag gcc aag ctg gat aag gac gga atg tat cca    672
   Leu Asn Pro Ile Ser Lys Ala Lys Leu Asp Lys Asp Gly Met Tyr Pro
       210                 215                 220
   gtt gaa atc tgg cat cca gat cca gca aaa aat gag aac aca agg tac    720
   Val Glu Ile Trp His Pro Asp Pro Ala Lys Asn Glu Asn Thr Arg Tyr
   225                 230                 235                 240
   ttt ggc aat tac act gga ggc acg tgc acc cca ccc gtc ctg cag ttc     768
   Phe Gly Asn Tyr Thr Gly Gly Thr Cys Thr Pro Pro Val Leu Gln Phe
                   245                 250                 255
   aca aac acc ctg aca act gtg ctc cta gat gaa aat gga gtt ggg ccc     816
   Thr Asn Thr Leu Thr Thr Val Leu Leu Asp Glu Asn Gly Val Gly Pro
               260                 265                 270
   ctc agc aaa gga gaa ggt cta tac ctc tcg agc gta gat ata atg ggc     864
   Leu Ser Lys Gly Glu Gly Leu Tyr Leu Ser Ser Val Asp Ile Met Gly
           275                 280                 285
   tgg aga gtt aca aga aac tat gat gtc cat cac tgg aga ggg ctt ccc     912
   Trp Arg Val Thr Arg Asn Tyr Asp Val His His Trp Arg Gly Leu Pro
       290                 295                 300
   aga tat ttc aaa atc acc ctg aga aaa aga tgg gtc aaa aat ccc tat     960
   Arg Tyr Phe Lys Ile Thr Leu Arg Lys Arg Trp Val Lys Asn Pro Tyr
   305                 310                 315                 320
   ccc atg gcc tcc ctc ata agt tcc ctt ttc aac aac atg ctc ccc caa    1008
   Pro Met Ala Ser Leu Ile Ser Ser Leu Phe Asn Asn Met Leu Pro Gln
                   325                 330                 335
   gtg cag ggc caa ccc atg gaa ggg gag aac acc cag gta gag gag gtt    1056
   Val Gln Gly Gln Pro Met Glu Gly Glu Asn Thr Gln Val Glu Glu Val
               340                 345                 350
   aga gtg tat gat ggg act gaa cct gta ccg ggg gac cct gat atg acg    1104
   Arg Val Tyr Asp Gly Thr Glu Pro Val Pro Gly Asp Pro Asp Met Thr
           355                 360                 365
   cgc tat gtt gac cgc ttt gga aaa aca aag act gta ttt cct ccc ggg    1152
   Arg Tyr Val Asp Arg Phe Gly Lys Thr Lys Thr Val Phe Pro Pro Gly
       370                 375                 380
<210> 2
   <211> 384
   <212> PRT
   <213> Künstliche Sequenz
   <223> Beschreibung der künstlichen Sequenz: Variante von Polyomavirus VP1 mit allen natürlichen Cysteinen ersetzt durch Serin, und Einführen eines neuen Cysteins anstelle von Thr 249 (PyVP1-Calls-T249C)
<400> 2
   Met Ala Pro Lys Arg Lys Ser Gly Val Ser Lys Ser Glu Thr Lys Ser
   1 5 10 15
   Thr Lys Ala Ser Pro Arg Pro Ala Pro Val Pro Lys Leu Leu Ile Lys
   20 25 30
   Gly Gly Met Glu Val Leu Asp Leu Val Thr Gly Pro Asp Ser Val Thr
   35 40 45
   Glu Ile Glu Ala Phe Leu Asn Pro Arg Met Gly Gln Pro Pro Thr Pro
   50 55 60
   Glu Ser Leu Thr Glu Gly Gly Gln Tyr Tyr Gly Trp Ser Arg Gly Ile
   65 70 75 80
   Asn Leu Ala Thr Ser Asp Thr Glu Asp Ser Pro Gly Asn Asn Thr Leu
   85 90 95
   Pro Thr Trp Ser Met Ala Lys Leu Gln Leu Pro Met Leu Asn Glu Asp
   100 105 110
   Leu Thr Ser Asp Thr Leu Gln Met Trp Glu Ala Val Ser Val Lys Thr
   115 120 125
   Glu Val Val Gly Ser Gly Ser Leu Leu Asp Val His Gly Phe Asn Lys
   130 135 140
   Pro Thr Asp Thr Val Asn Thr Lys Gly Ile Ser Thr Pro Val Glu Gly
   145 150 155 160
   Ser Gln Tyr His Val Phe Ala Val Gly Gly Glu Pro Leu Asp Leu Gln
   165 170 175
   Gly Leu Val Thr Asp Ala Arg Thr Lys Tyr Lys Glu Glu Gly Val Val
   180 185 190
   Thr Ile Lys Thr Ile Thr Lys Lys Asp Met Val Asn Lys Asp Gln Val
   195 200 205
   Leu Asn Pro Ile Ser Lys Ala Lys Leu Asp Lys Asp Gly Met Tyr Pro
   210 215 220
   Val Glu Ile Trp His Pro Asp Pro Ala Lys Asn Glu Asn Thr Arg Tyr
   225 230 235 240
   Phe Gly Asn Tyr Thr Gly Gly Thr Cys Thr Pro Pro Val Leu Gln Phe
   245 250 255
   Thr Asn Thr Leu Thr Thr Val Leu Leu Asp Glu Asn Gly Val Gly Pro
   260 265 270
   Leu Ser Lys Gly Glu Gly Leu Tyr Leu Ser Ser Val Asp Ile Met Gly
   275 280 285
   Trp Arg Val Thr Arg Asn Tyr Asp Val His His Trp Arg Gly Leu Pro
   290 295 300
   Arg Tyr Phe Lys Ile Thr Leu Arg Lys Arg Trp Val Lys Asn Pro Tyr
   305 310 315 320
   Pro Met Ala Ser Leu Ile Ser Ser Leu Phe Asn Asn Met Leu Pro Gln
   325 330 335
   Val Gln Gly Gln Pro Met Glu Gly Glu Asn Thr Gln Val Glu Glu Val
   340 345 350
   Arg Val Tyr Asp Gly Thr Glu Pro Val Pro Gly Asp Pro Asp Met Thr
   355 360 365
   Arg Tyr Val Asp Arg Phe Gly Lys Thr Lys Thr Val Phe Pro Pro Gly
   370 375 380
<210> 3
   <211> 963
   <212> DNA
   <213> Künstliche Sequenz
<220>
   <223> Beschreibung der künstlichen Sequenz: VP1-Protein von Polyomavirus, verkürzt um 63 Aminosäuren am C-Terminus, Ersetzung aller Cysteine durch Serin sowie Austausch von Thr 249 gegen Cys (PyVPl-DCT63)
<220>
   <221> CDS
   <222> (1)..(963)
<400> 3
   atg gcc ccc aaa aga aaa agc ggc gtc tct aaa agc gag aca aaa agc 48
   Met Ala Pro Lys Arg Lys Ser Gly Val Ser Lys Ser Glu Thr Lys Ser
   1 5 10 15
   aca aag gct agc cca aga ccc gca ccc gtt ccc aaa ctg ctt att aaa 96
   Thr Lys Ala Ser Pro Arg Pro Ala Pro Val Pro Lys Leu Leu Ile Lys
               20                  25                  30
   ggg ggt atg gag gtg ctg gac ctt gtg aca ggg cca gac agt gtg aca     144
   Gly Gly Met Glu Val Leu Asp Leu Val Thr Gly Pro Asp Ser Val Thr
           35                  40                  45
   gaa ata gaa gct ttt ctg aac ccc aga atg ggg cag cca ccc acc cct     192
   Glu Ile Glu Ala Phe Leu Asn Pro Arg Met Gly Gln Pro Pro Thr Pro
       50                  55                  60
   gaa agc cta aca gag gga ggg caa tac tat ggt tgg agc aga ggg att     240
   Glu Ser Leu Thr Glu Gly Gly Gln Tyr Tyr Gly Trp Ser Arg Gly Ile
   65                  70                  75                  80
   aat ttg gct aca tca gat aca gag gat tcc cca gga aat aat aca ctt     288
   Asn Leu Ala Thr Ser Asp Thr Glu Asp Ser Pro Gly Asn Asn Thr Leu
                   85                  90                  95
   ccc aca tgg agt atg gca aag ctc cag ctt ccc atg ctc aat gag gac     336
   Pro Thr Trp Ser Met Ala Lys Leu Gln Leu Pro Met Leu Asn Glu Asp
               100                 105                 110
   ctc acg tct gac acc cta caa atg tgg gag gca gtc tca gtg aaa acc     384
   Leu Thr Ser Asp Thr Leu Gln Met Trp Glu Ala Val Ser Val Lys Thr
           115                 120                 125
   gag gtg gtg ggc tct ggc tca ctg tta gat gtg cat ggg ttc aac aaa     432
   Glu Val Val Gly Ser Gly Ser Leu Leu Asp Val His Gly Phe Asn Lys
       130                 135                 140
   ccc aca gat aca gta aac aca aaa gga att tcc act cca gtg gaa ggc     480
   Pro Thr Asp Thr Val Asn Thr Lys Gly Ile Ser Thr Pro Val Glu Gly
   145                 150                 155                 160
   agc caa tat cat gtg ttt gct gtg ggc ggg gaa ccg ctt gac ctc cag     528
   Ser Gln Tyr His Val Phe Ala Val Gly Gly Glu Pro Leu Asp Leu Gln
                   165                 170                 175
   gga ctt gtg aca gat gcc aga aca aaa tac aag gaa gaa ggg gta gta     576
   Gly Leu Val Thr Asp Ala Arg Thr Lys Tyr Lys Glu Glu Gly Val Val
               180                 185                 190
   aca atc aaa aca atc aca aag aag gac atg gtc aac aaa gac caa gtc     624
   Thr Ile Lys Thr Ile Thr Lys Lys Asp Met Val Asn Lys Asp Gln Val
           195                 200                 205
   ctg aat cca att agc aag gcc aag ctg gat aag gac gga atg tat cca     672
   Leu Asn Pro Ile Ser Lys Ala Lys Leu Asp Lys Asp Gly Met Tyr Pro
       210                 215                 220
   gtt gaa atc tgg cat cca gat cca gca aaa aat gag aac aca agg tac     720
   Val Glu Ile Trp His Pro Asp Pro Ala Lys Asn Glu Asn Thr Arg Tyr
   225                 230                 235                 240
   ttt ggc aat tac act gga ggc acg tgc acc cca ccc gtc ctg cag ttc     768
   Phe Gly Asn Tyr Thr Gly Gly Thr Cys Thr Pro Pro Val Leu Gln Phe
                   245                 250                 255
   aca aac acc ctg aca act gtg ctc cta gat gaa aat gga gtt ggg ccc     816
   Thr Asn Thr Leu Thr Thr Val Leu Leu Asp Glu Asn Gly Val Gly Pro
               260                 265                 270
   ctc agc aaa gga gaa ggt cta tac ctc tcg agc gta gat ata atg ggc     864
   Leu Ser Lys Gly Glu Gly Leu Tyr Leu Ser Ser Val Asp Ile Met Gly
           275                 280                 285
   tgg aga gtt aca aga aac tat gat gtc cat cac tgg aga ggg ctt ccc     912
   Trp Arg Val Thr Arg Asn Tyr Asp Val His His Trp Arg Gly Leu Pro
       290                 295                 300
   aga tat ttc aaa atc acc ctg aga aaa aga tgg gtc aaa aat ccc tat     960
   Arg Tyr Phe Lys Ile Thr Leu Arg Lys Arg Trp Val Lys Asn Pro Tyr
   305                 310                 315                 320
   ccc                                                                 963
   Pro
<210> 4
   <211> 321
   <212> PRT
   <213> Künstliche Sequenz
   <223> Beschreibung der künstlichen Sequenz: VP1-Protein von Polyomavirus, verkürzt um 63 Aminosäuren am C-Terminus, Ersetzung aller Cysteine durch Serin sowie Austausch von Thr 249 gegen Cys (PyVP1-DCT63)
<400> 4
   Met Ala Pro Lys Arg Lys Ser Gly Val Ser Lys Ser Glu Thr Lys Ser
    1               5                  10                  15
   Thr Lys Ala Ser Pro Arg Pro Ala Pro Val Pro Lys Leu Leu Ile Lys
               20                  25                  30
   Gly Gly Met Glu Val Leu Asp Leu Val Thr Gly Pro Asp Ser Val Thr
   35 40 45
   Glu Ile Glu Ala Phe Leu Asn Pro Arg Met Gly Gln Pro Pro Thr Pro
   50 55 60
   Glu Ser Leu Thr Glu Gly Gly Gln Tyr Tyr Gly Trp Ser Arg Gly Ile
   65 70 75 80
   Asn Leu Ala Thr Ser Asp Thr Glu Asp Ser Pro Gly Asn Asn Thr Leu
   85 90 95
   Pro Thr Trp Ser Met Ala Lys Leu Gln Leu Pro Met Leu Asn Glu Asp
   100 105 110
   Leu Thr Ser Asp Thr Leu Gln Met Trp Glu Ala Val Ser Val Lys Thr
   115 120 125
   Glu Val Val Gly Ser Gly Ser Leu Leu Asp Val His Gly Phe Asn Lys
   130 135 140
   Pro Thr Asp Thr Val Asn Thr Lys Gly Ile Ser Thr Pro Val Glu Gly
   145 150 155 160
   Ser Gln Tyr His Val Phe Ala Val Gly Gly Glu Pro Leu Asp Leu Gln
   165 170 175
   Gly Leu Val Thr Asp Ala Arg Thr Lys Tyr Lys Glu Glu Gly Val Val
   180 185 190
   Thr Ile Lys Thr Ile Thr Lys Lys Asp Met Val Asn Lys Asp Gln Val
   195 200 205
   Leu Asn Pro Ile Ser Lys Ala Lys Leu Asp Lys Asp Gly Met Tyr Pro
   210 215 220
   Val Glu Ile Trp His Pro Asp Pro Ala Lys Asn Glu Asn Thr Arg Tyr
   225 230 235 240
   Phe Gly Asn Tyr Thr Gly Gly Thr Cys Thr Pro Pro Val Leu Gln Phe
   245 250 255
   Thr Asn Thr Leu Thr Thr Val Leu Leu Asp Glu Asn Gly Val Gly Pro
   260 265 270
   Leu Ser Lys Gly Glu Gly Leu Tyr Leu Ser Ser Val Asp Ile Met Gly
   275 280 285
   Trp Arg Val Thr Arg Asn Tyr Asp Val His His Trp Arg Gly Leu Pro
   290 295 300
   Arg Tyr Phe Lys Ile Thr Leu Arg Lys Arg Trp Val Lys Asn Pro Tyr
   305 310 315 320
   Pro
<210> 5
   <211> 42
   <212> PRT
   <213> Homo sapiens
<220>
   <221> PEPTIDE
   <222> (1)..(42)
   <223> Beschreibung: Alzheimer-Beta-Peptid (1-42)
<400> 5